# NEW EUROPEAN PATENT SPECIFICATION

(11) **EP 2 821 459 B2**
(45) Date of publication and mention of the opposition decision: **12.08.2026**
(45) Mention of the grant of the patent: 04.10.2017
(21) Application number: 13196046.0
(22) Date of filing: 06.12.2013
(51) Int. Cl.: C09K 11/06, C07D 209/82, H05B 33/20, C07D 405/04, C07D 409/04, C07D 487/04, C07D 209/86, H10K 85/60, H10K 101/10, H10K 101/00, H10K 50/11

(54) **COMPOSITION AND ORGANIC OPTOELECTRIC DEVICE AND DISPLAY DEVICE**
Zusammensetzung und organische optoelektrische Vorrichtung und Anzeigevorrichtung
Composition et dispositif optoélectronique organique et dispositif d'affichage

(30) Priority: 01.07.2013 KR 20130076669
(43) Date of publication of application: 07.01.2015
(73) Proprietor: Cheil Industries Inc., Gumi-si Gyeongsangbuk-do 730-030 (KR)
(72) Inventor: Min, Soo-Hyun, Gyeonggi-do 437-711 (KR); Kang, Gi-Wook, Gyeonggi-do 437-711 (KR); Kang, Dong-Min, Gyeonggi-do 437-711 (KR); Kang, Eui-Su, Gyeonggi-do 437-711 (KR); Kim, Youn-Hwan, Gyeonggi-do 437-711 (KR); Yang, Yong-Tak, Gyeonggi-do 437-711 (KR); Oh, Jae-Jin, Gyeonggi-do 437-711 (KR); Lee, Nam-Heon, Gyeonggi-do 437-711 (KR); Lui, Jin-Hyun, Gyeonggi-do 437-711 (KR); Yu, Eun-Sun, Gyeonggi-do 437-711 (KR); Jung, Ho-Kuk, Gyeonggi-do 437-711 (KR); Jo, Young-Kyoung, Gyeonggi-do 437-711 (KR)
(74) Representative: Michalski Hüttermann & Partner mbB

(56) References cited:
- EP-A1- 2 403 028
- EP-A1- 2 709 181
- WO-A1-2011/152596
- WO-A1-2012/087955
- JP-A- 2003 133 075
- JP-A- 2007 134 677
- KR-A- 20100 028 471
- US-A1- 2010 187 977
- US-A1- 2012 001 165
- US-A1- 2012 223 295
- US-A1- 2013 150 576
- US-A1- 2014 217 378
- US-A1- 2014 299 865
- US-A1- 2014 306 207

## Description

This application claims priority to and the benefit of Korean Patent Application No. 10-2013-0076669 filed in the Korean Intellectual Property Office on July 1, 2013.

### BACKGROUND OF THE INVENTION

### (a) Field of the Invention

A composition, an organic optoelectric device, and a display device are disclosed.

### (b) Description of the Related Art

An organic optoelectric device is a device that converts electrical energy into photoenergy, and vice versa.

An organic optoelectric device may be classified as follows in accordance with its driving principles.

One is an electronic device where excitons generated by photoenergy are separated into electrons and holes and the electrons and holes are transferred to separate electrodes respectively and electrical energy is produced, and the other is a light emitting device to generate photoenergy from electrical energy by supplying a voltage or a current to electrodes.

Examples of the organic optoelectric device may include an organic photoelectric device, an organic light emitting diode, an organic solar cell, and an organic photo-conductor drum, and the like.

Among them, the organic light emitting diode (OLED) has recently drawn attention due to an increase in demand for flat panel displays.

The organic light emitting diode converts electrical energy into light by applying current to an organic light emitting material, and has a structure in which an organic layer is interposed between an anode and a cathode.

Herein, the organic layer may include an emission layer and optionally an auxiliary layer, and the auxiliary layer may include at least one selected from, for example a hole injection layer, a hole transport layer, an electron blocking layer, an electron transport layer, an electron injection layer, and a hole blocking layer in order to improve efficiency and stability of an organic light emitting diode.

Performance of an organic light emitting diode may be affected by characteristics of the organic layer, and among them, may be mainly affected by characteristics of an organic material of the organic layer.

Particularly, development for an organic material being capable of increasing hole and electron mobility and simultaneously increasing electrochemical stability is needed so that the organic light emitting diode may be applied to a large-size flat panel display.

Compounds for optoelectronic devices are inter alia disclosed in the US 2012/001165, EP 2 403 028, WO 2012/087955, US 2013/150576, US 2012/223295, KR 2010/ 0028471 and WO 2011/152596.

### SUMMARY OF THE INVENTION

One embodiment provides a composition capable of realizing an organic optoelectric device having high efficiency and long life-span.

Another embodiment provides an organic optoelectric device including the composition.

Yet another embodiment provides a display device including the organic optoelectric device.

According to one embodiment, provided is a composition including a first host compound including moieties represented by the following Chemical Formulae 1 to 3 that are sequentially bonded with each other and a second host compound including at least one carbazole group with a substituent having hole characteristics:

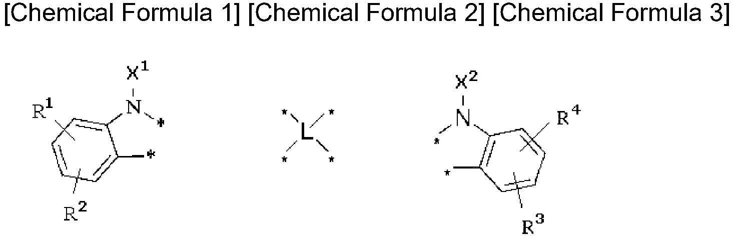

In the Chemical Formulae 1 to 3,
X¹ is *-Y¹-ET,
X² is *-Y²-Ar¹,
ET is a substituent capable of accepting an electron when electric field is applied,
Ar¹ is a substituted or unsubstituted C6 to C30 aryl group, a substituted or unsubstituted C2 to C30 heteroaryl group, or a combination thereof,
Y' and Y² are each independently a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof,
L is a substituted or unsubstituted C2 or C3 alkenylene group or a substituted or unsubstituted C6 to C20 arylene group, and
R¹ to R⁴ are each independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

According to another embodiment, an organic optoelectric device includes an anode and a cathode facing each other, at least one organic layer interposed between the anode and the cathode, wherein the organic layer includes the composition.

According to yet another embodiment, a display device including the organic optoelectric device is provided.

An organic optoelectric device having high efficiency and long life-span may be realized.

### BRIEF DESCRIPTION OF THE DRAWINGS

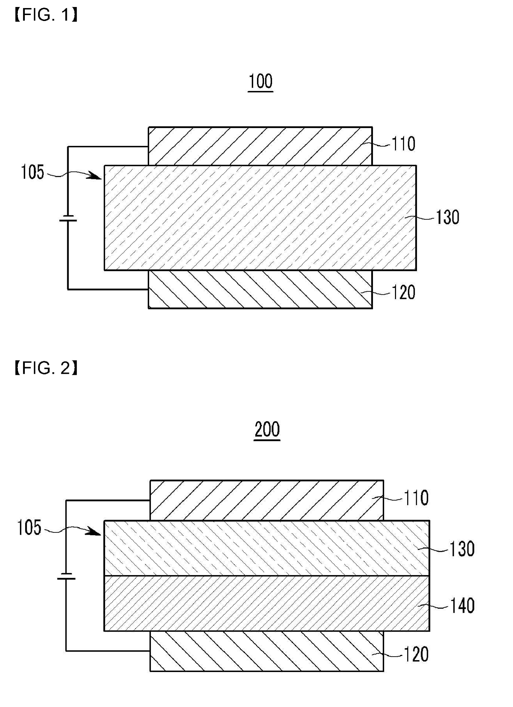

FIGS. 1 and 2 are cross-sectional views of each organic light emitting diode according to one embodiment.

### DETAILED DESCRIPTION

Hereinafter, embodiments of the present invention are described in detail.

However, these embodiments are exemplary, and this disclosure is not limited thereto.

As used herein, when a definition is not otherwise provided, the term "substituted" refers to one substituted with a deuterium, a halogen, a hydroxy group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C3 to C30 cycloalkyl group, a C3 to C30 heterocycloalkyl group, a C6 to C30 aryl group, a C6 to C30 heteroaryl group, a C1 to C20 alkoxy group, a fluoro group, a C1 to C10 trifluoroalkyl group such as a trifluoromethyl group, and the like, or a cyano group, instead of at least one hydrogen of a substituent or a compound.

In addition, two adjacent substituents of the substituted halogen, hydroxy group, an amino group, a substituted or unsubstituted C1 to C20 amine group, a nitro group, a substituted or unsubstituted C3 to C40 silyl group, C1 to C30 alkyl group, C1 to C10 alkylsilyl group, C3 to C30 cycloalkyl group, C3 to C30 heterocycloalkyl group, C6 to C30 aryl group, C6 to C30 heteroaryl group, C1 to C20 alkoxy group, fluoro group, C1 to C10 trifluoroalkyl group such as trifluoromethyl group and the like, or cyano group may be fused with each other to form a ring.

For example, the substituted C6 to C30 aryl group may be fused with another adjacent substituted C6 to C30 aryl group to form a substituted or unsubstituted fluorene ring.

In the present specification, when specific definition is not otherwise provided, the term "hetero" refers to one including 1 to 3 hetero atoms selected from N, O, S, P, and Si, and remaining carbons in one compound or substituent.

In the present specification, when a definition is not otherwise provided, the term "alkyl group" may refer to an aliphatic hydrocarbon group.

The alkyl group may refer to "a saturated alkyl" without any double bond or triple bond.

The alkyl group may be a C1 to C30 alkyl group. More specifically, the alkyl group may be a C1 to C20 alkyl group or a C1 to C10 alkyl group. For example, a C1 to C4 alkyl group includes 1 to 4 carbon in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

In the present specification, the term "aryl group" refers to a substituent including all element of the cycle having p-orbitals which form conjugation, and may be monocyclic, polycyclic or fused ring polycyclic (i.e., rings sharing adjacent pairs of carbon atoms) functional group.

As used herein, the term "heteroaryl group" may refer to aryl group including at least one hetero atoms selected from N, O, S, P, and Si and remaining carbons in one functional group.

The heteroaryl group may be a fused ring where each ring may include the 1 to 3 heteroatoms.

More specifically, the substituted or unsubstituted C6 to C30 aryl group and/or the substituted or unsubstituted C2 to C30 heteroaryl group may be a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted fluorenyl group, a substituted or unsubstituted dibenzothiophenyl group, a substituted or unsubstituted dibenzofuranyl group, a substituted or unsubstituted carbazole group, or a combination thereof, is not limited thereto.

In the specification, hole characteristics refer to characteristics capable of donating an electron to form a hole when electric field is applied, and characteristics that hole formed in the anode is easily injected into the emission layer and transported in the emission layer due to conductive characteristics according to HOMO level.

In addition, electron characteristics refer to characteristics capable of accepting an electron when electric field is applied, and characteristics that electron formed in the cathode is easily injected into the emission layer and transported in the emission layer due to conductive characteristics according to LUMO level.

Hereinafter, a composition according to one embodiment is described.

A composition according to one embodiment includes a first host compound having electron characteristics and a second host compound having hole characteristics.

The first host compound includes a substituent having characteristics capable of accepting an electron when electric field is applied, that is, electron characteristics, and includes moieties represented by the Chemical Formulae 1 to 3 that are sequentially bonded with each other.

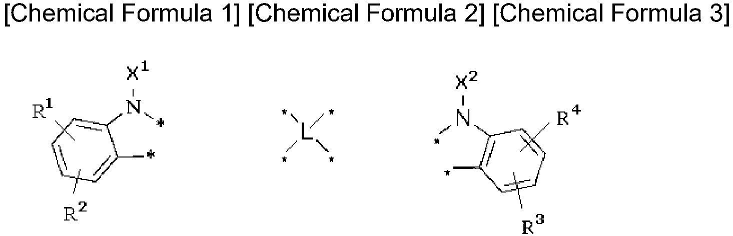

In the Chemical Formulae 1 to 3,
X¹ is *-Y¹-ET,
X² is *-Y²-Ar¹,
ET is a substituent capable of accepting an electron when electric field is applied,
Ar¹ is a substituted or unsubstituted C6 to C30 aryl group, a substituted or unsubstituted C2 to C30 heteroaryl group, or a combination thereof,
Y¹ and Y² are each independently a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof,
L is a substituted or unsubstituted C2 or C3 alkenylene group or a substituted or unsubstituted C6 to C20 arylene group, and
R¹ to R⁴ are each independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

In the Chemical Formulae 1 to 3, "*" indicates linking points between Chemical Formula 1 and Chemical Formula 2 and between Chemical Formula 2 and Chemical Formula 3.

Herein, ET may be, for example a substituent capable of transporting an electron, and may be, for example a substituted or unsubstituted pyridinyl group, a substituted or unsubstituted pyrimidinyl group, a substituted or unsubstituted triazinyl group, a substituted or unsubstituted pyrazinyl group, a substituted or unsubstituted pyridazinyl group, a substituted or unsubstituted purinyl group, a substituted or unsubstituted quinolinyl group, a substituted or unsubstituted isoquinolinyl group, a substituted or unsubstituted phthalazinyl group, a substituted or unsubstituted naphpyridinyl group, a substituted or unsubstituted quinoxalinyl group, a substituted or unsubstituted quinazolinyl group, a substituted or unsubstituted acridinyl group, a substituted or unsubstituted phenanthrolinyl group, a substituted or unsubstituted phenazinyl group, a substituted or unsubstituted imidazolyl group, a substituted or unsubstituted triazolyl group, a substituted or unsubstituted tetrazolyl group, a substituted or unsubstituted oxadiazolyl group, a substituted or unsubstituted oxatriazolyl group, a substituted or unsubstituted thiatriazolyl group, a substituted or unsubstituted benzimidazolyl group, a substituted or unsubstituted benzotriazolyl group, or a combination thereof.

The ET may be a heteroaryl group including at least one nitrogen atom, except for a carbazolyl group, and may be represented by, for example the following Chemical Formula 1a.

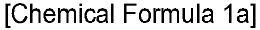

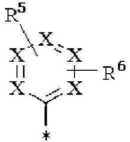

In the Chemical Formula 1a,
X is N, C or CR^{a},
at least one of X is N, and
R⁵, R⁶ and R^{a} are each independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

The ET may be one of the substituents listed in the Group 1.

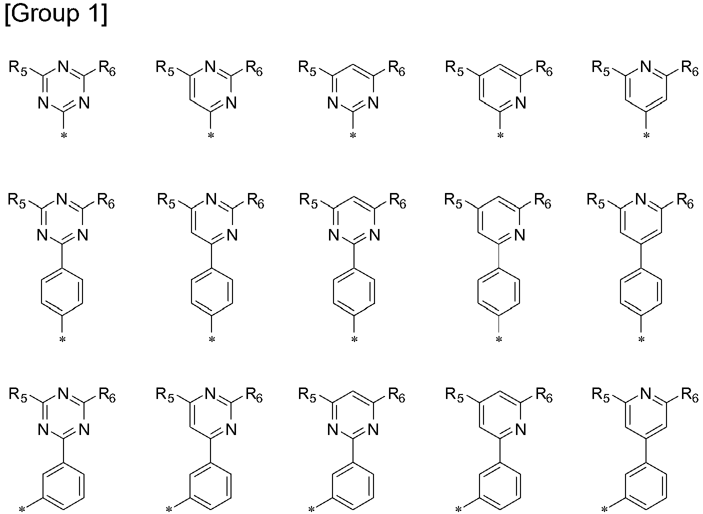

In the Group 1, R⁵ and R⁶ are the same as described above.

The X¹ may be, for example one of the substituents listed in the Group 2.

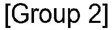

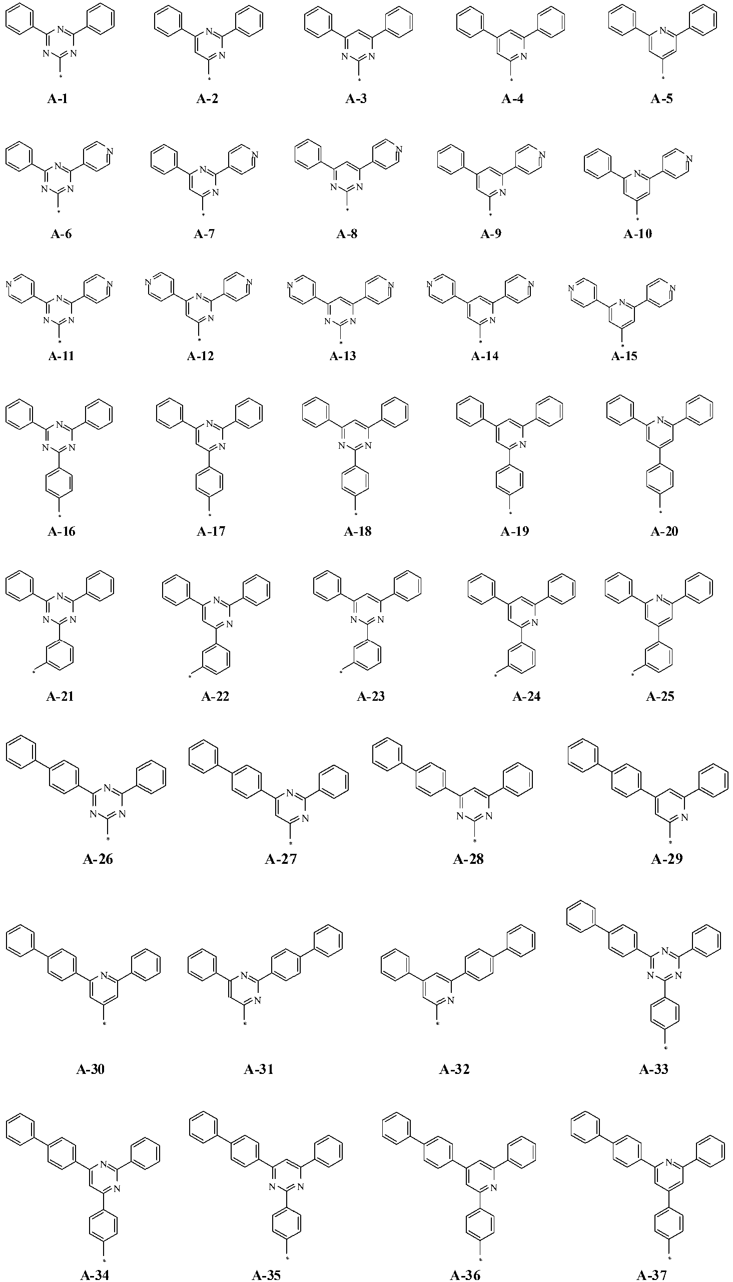

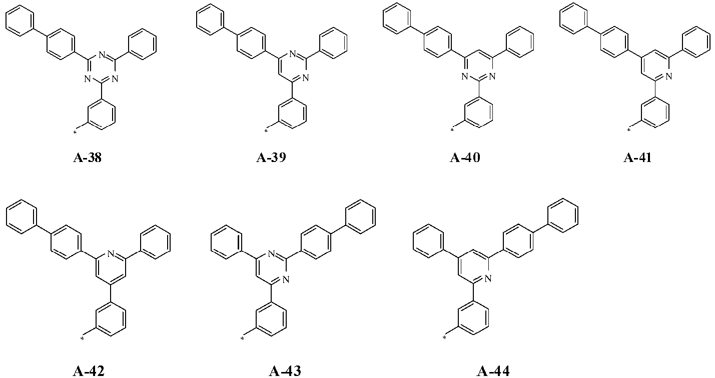

The moiety represented by the Chemical Formula 2 may be, for example represented by one of the Chemical Formulae 2-1 to 2-3, but is not limited thereto.

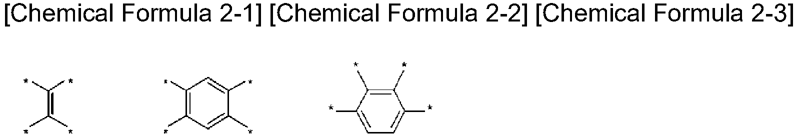

In the moiety represented by the Chemical Formula 3, Ar¹ may be, for example a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted carbazolyl group, a substituted or unsubstituted benzofuranyl group, a substituted or unsubstituted benzothiophenyl group, a substituted or unsubstituted fluorenyl group, a substituted or unsubstituted dibenzofuranyl group, a substituted or unsubstituted dibenzothiophenyl group, or a combination thereof.

The X² may be, for example one of the substituents listed in the Group 3, but is not limited thereto.

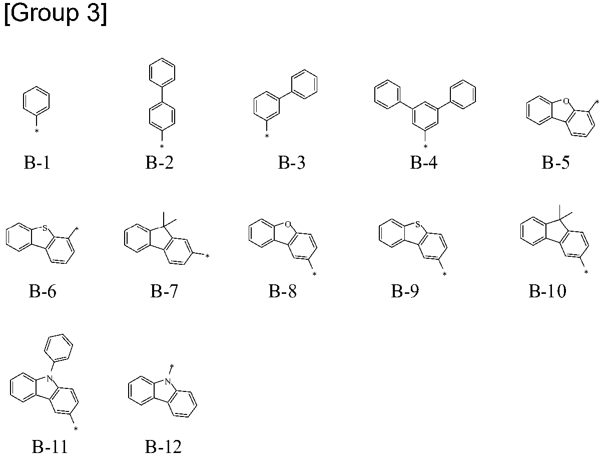

The first host compound may be, for example one of compounds listed in the Group 4, but is not limited thereto.

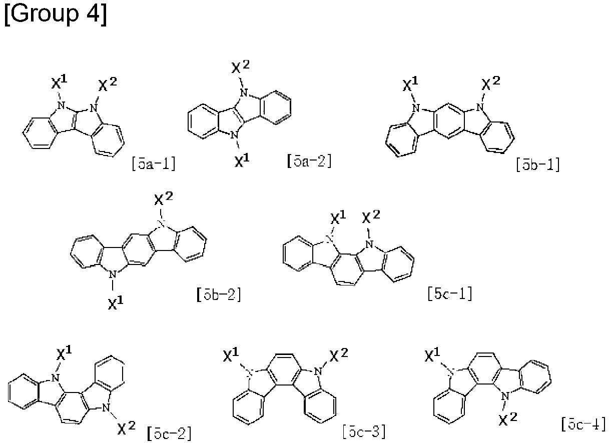

In the Group 4, X¹ and X² are the same as described above.

The first host compound may be, for example compounds listed in the Tables 1 to 11.

**[Table 1]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-1 | 5a-1 | A-1 | B-1 | 1-47 | 5a-1 | A-3 | B-2 | 1-93 | 5a-1 | A-5 | B-3 |
| 1-2 | 5a-1 | A-2 | B-1 | 1-48 | 5a-1 | A-4 | B-2 | 1-94 | 5a-1 | A-6 | B-3 |
| 1-3 | 5a-1 | A-3 | B-1 | 1-49 | 5a-1 | A-5 | B-2 | 1-95 | 5a-1 | A-7 | B-3 |
| 1-4 | 5a-1 | A-4 | B-1 | 1-50 | 5a-1 | A-6 | B-2 | 1-96 | 5a-1 | A-8 | B-3 |
| 1-5 | 5a-1 | A-5 | B-1 | 1-51 | 5a-1 | A-7 | B-2 | 1-97 | 5a-1 | A-9 | B-3 |
| 1-6 | 5a-1 | A-6 | B-1 | 1-52 | 5a-1 | A-8 | B-2 | 1-98 | 5a-1 | A-10 | B-3 |
| 1-7 | 5a-1 | A-7 | B-1 | 1-53 | 5a-1 | A-9 | B-2 | 1-99 | 5a-1 | A-11 | B-3 |
| 1-8 | 5a-1 | A-8 | B-1 | 1-54 | 5a-1 | A-10 | B-2 | 1-100 | 5a-1 | A-12 | B-3 |
| 1-9 | 5a-1 | A-9 | B-1 | 1-55 | 5a-1 | A-11 | B-2 | 1-101 | 5a-1 | A-13 | B-3 |
| 1-10 | 5a-1 | A-10 | B-1 | 1-56 | 5a-1 | A-12 | B-2 | 1-102 | 5a-1 | A-14 | B-3 |
| 1-11 | 5a-1 | A-11 | B-1 | 1-57 | 5a-1 | A-13 | B-2 | 1-103 | 5a-1 | A-15 | B-3 |
| 1-12 | 5a-1 | A-12 | B-1 | 1-58 | 5a-1 | A-14 | B-2 | 1-104 | 5a-1 | A-16 | B-3 |
| 1-13 | 5a-1 | A-13 | B-1 | 1-59 | 5a-1 | A-15 | B-2 | 1-105 | 5a-1 | A-17 | B-3 |
| 1-14 | 5a-1 | A-14 | B-1 | 1-60 | 5a-1 | A-16 | B-2 | 1-106 | 5a-1 | A-18 | B-3 |
| 1-15 | 5a-1 | A-15 | B-1 | 1-61 | 5a-1 | A-17 | B-2 | 1-107 | 5a-1 | A-19 | B-3 |
| 1-16 | 5a-1 | A-16 | B-1 | 1-62 | 5a-1 | A-18 | B-2 | 1-108 | 5a-1 | A-20 | B-3 |
| 1-17 | 5a-1 | A-17 | B-1 | 1-63 | 5a-1 | A-19 | B-2 | 1-109 | 5a-1 | A-21 | B-3 |
| 1-18 | 5a-1 | A-18 | B-1 | 1-64 | 5a-1 | A-20 | B-2 | 1-110 | 5a-1 | A-22 | B-3 |
| 1-19 | 5a-1 | A-19 | B-1 | 1-65 | 5a-1 | A-21 | B-2 | 1-111 | 5a-1 | A-23 | B-3 |
| 1-20 | 5a-1 | A-20 | B-1 | 1-66 | 5a-1 | A-22 | B-2 | 1-112 | 5a-1 | A-24 | B-3 |
| 1-21 | 5a-1 | A-21 | B-1 | 1-67 | 5a-1 | A-23 | B-2 | 1-113 | 5a-1 | A-25 | B-3 |
| 1-22 | 5a-1 | A-22 | B-1 | 1-68 | 5a-1 | A-24 | B-2 | 1-114 | 5a-1 | A-26 | B-3 |
| 1-23 | 5a-1 | A-23 | B-1 | 1-69 | 5a-1 | A-25 | B-2 | 1-115 | 5a-1 | A-27 | B-3 |
| 1-24 | 5a-1 | A-24 | B-1 | 1-70 | 5a-1 | A-26 | B-2 | 1-116 | 5a-1 | A-28 | B-3 |
| 1-25 | 5a-1 | A-25 | B-1 | 1-71 | 5a-1 | A-27 | B-2 | 1-117 | 5a-1 | A-29 | B-3 |
| 1-26 | 5a-1 | A-26 | B-1 | 1-72 | 5a-1 | A-28 | B-2 | 1-118 | 5a-1 | A-30 | B-3 |
| 1-27 | 5a-1 | A-27 | B-1 | 1-73 | 5a-1 | A-29 | B-2 | 1-119 | 5a-1 | A-31 | B-3 |
| 1-28 | 5a-1 | A-28 | B-1 | 1-74 | 5a-1 | A-30 | B-2 | 1-120 | 5a-1 | A-32 | B-3 |
| 1-29 | 5a-1 | A-29 | B-1 | 1-75 | 5a-1 | A-31 | B-2 | 1-121 | 5a-1 | A-33 | B-3 |
| 1-30 | 5a-1 | A-30 | B-1 | 1-76 | 5a-1 | A-32 | B-2 | 1-122 | 5a-1 | A-34 | B-3 |
| 1-31 | 5a-1 | A-31 | B-1 | 1-77 | 5a-1 | A-33 | B-2 | 1-123 | 5a-1 | A-35 | B-3 |
| 1-32 | 5a-1 | A-32 | B-1 | 1-78 | 5a-1 | A-34 | B-2 | 1-124 | 5a-1 | A-36 | B-3 |
| 1-33 | 5a-1 | A-33 | B-1 | 1-79 | 5a-1 | A-35 | B-2 | 1-125 | 5a-1 | A-37 | B-3 |
| 1-34 | 5a-1 | A-34 | B-1 | 1-80 | 5a-1 | A-36 | B-2 | 1-126 | 5a-1 | A-38 | B-3 |
| 1-35 | 5a-1 | A-35 | B-1 | 1-81 | 5a-1 | A-37 | B-2 | 1-127 | 5a-1 | A-39 | B-3 |
| 1-36 | 5a-1 | A-36 | B-1 | 1-82 | 5a-1 | A-38 | B-2 | 1-128 | 5a-1 | A-40 | B-3 |
| 1-37 | 5a-1 | A-37 | B-1 | 1-83 | 5a-1 | A-39 | B-2 | 1-129 | 5a-1 | A-41 | B-3 |
| 1-38 | 5a-1 | A-38 | B-1 | 1-84 | 5a-1 | A-40 | B-2 | 1-130 | 5a-1 | A-42 | B-3 |
| 1-39 | 5a-1 | A-39 | B-1 | 1-85 | 5a-1 | A-41 | B-2 | 1-131 | 5a-1 | A-43 | B-3 |
| 1-40 | 5a-1 | A-40 | B-1 | 1-86 | 5a-1 | A-42 | B-2 | 1-132 | 5a-1 | A-44 | B-3 |
| 1-41 | 5a-1 | A-41 | B-1 | 1-87 | 5a-1 | A-43 | B-2 | 1-133 | 5a-1 | A-1 | B-4 |
| 1-42 | 5a-1 | A-42 | B-1 | 1-88 | 5a-1 | A-44 | B-3 | 1-134 | 5a-1 | A-2 | B-4 |
| 1-43 | 5a-1 | A-43 | B-1 | 1-89 | 5a-1 | A-1 | B-3 | 1-135 | 5a-1 | A-3 | B-4 |
| 1-44 | 5a-1 | A-44 | B-1 | 1-90 | 5a-1 | A-2 | B-3 | 1-136 | 5a-1 | A-4 | B-4 |
| 1-45 | 5a-1 | A-1 | B-2 | 1-91 | 5a-1 | A-3 | B-3 | 1-137 | 5a-1 | A-5 | B-4 |
| 1-46 | 5a-1 | A-2 | B-2 | 1-92 | 5a-1 | A-4 | B-3 | 1-138 | 5a-1 | A-6 | B-4 |

**[Table 2]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-139 | 5a-1 | A-7 | B-4 | 1-185 | 5a-2 | A-9 | B-1 | 1-231 | 5a-2 | A-11 | B-2 |
| 1-140 | 5a-1 | A-8 | B-4 | 1-186 | 5a-2 | A-10 | B-1 | 1-232 | 5a-2 | A-12 | B-2 |
| 1-141 | 5a-1 | A-9 | B-4 | 1-187 | 5a-2 | A-11 | B-1 | 1-233 | 5a-2 | A-13 | B-2 |
| 1-142 | 5a-1 | A-10 | B-4 | 1-188 | 5a-2 | A-12 | B-1 | 1-234 | 5a-2 | A-14 | B-2 |
| 1-143 | 5a-1 | A-11 | B-4 | 1-189 | 5a-2 | A-13 | B-1 | 1-235 | 5a-2 | A-15 | B-2 |
| 1-144 | 5a-1 | A-12 | B-4 | 1-190 | 5a-2 | A-14 | B-1 | 1-236 | 5a-2 | A-16 | B-2 |
| 1-145 | 5a-1 | A-13 | B-4 | 1-191 | 5a-2 | A-15 | B-1 | 1-237 | 5a-2 | A-17 | B-2 |
| 1-146 | 5a-1 | A-14 | B-4 | 1-192 | 5a-2 | A-16 | B-1 | 1-238 | 5a-2 | A-18 | B-2 |
| 1-147 | 5a-1 | A-15 | B-4 | 1-193 | 5a-2 | A-17 | B-1 | 1-239 | 5a-2 | A-19 | B-2 |
| 1-148 | 5a-1 | A-16 | B-4 | 1-194 | 5a-2 | A-18 | B-1 | 1-240 | 5a-2 | A-20 | B-2 |
| 1-149 | 5a-1 | A-17 | B-4 | 1-195 | 5a-2 | A-19 | B-1 | 1-241 | 5a-2 | A-21 | B-2 |
| 1-150 | 5a-1 | A-18 | B-4 | 1-196 | 5a-2 | A-20 | B-1 | 1-242 | 5a-2 | A-22 | B-2 |
| 1-151 | 5a-1 | A-19 | B-4 | 1-197 | 5a-2 | A-21 | B-1 | 1-243 | 5a-2 | A-23 | B-2 |
| 1-152 | 5a-1 | A-20 | B-4 | 1-198 | 5a-2 | A-22 | B-1 | 1-244 | 5a-2 | A-24 | B-2 |
| 1-153 | 5a-1 | A-21 | B-4 | 1-199 | 5a-2 | A-23 | B-1 | 1-245 | 5a-2 | A-25 | B-2 |
| 1-154 | 5a-1 | A-22 | B-4 | 1-200 | 5a-2 | A-24 | B-1 | 1-246 | 5a-2 | A-26 | B-2 |
| 1-155 | 5a-1 | A-23 | B-4 | 1-201 | 5a-2 | A-25 | B-1 | 1-247 | 5a-2 | A-27 | B-2 |
| 1-156 | 5a-1 | A-24 | B-4 | 1-202 | 5a-2 | A-26 | B-1 | 1-248 | 5a-2 | A-28 | B-2 |
| 1-157 | 5a-1 | A-25 | B-4 | 1-203 | 5a-2 | A-27 | B-1 | 1-249 | 5a-2 | A-29 | B-2 |
| 1-158 | 5a-1 | A-26 | B-4 | 1-204 | 5a-2 | A-28 | B-1 | 1-250 | 5a-2 | A-30 | B-2 |
| 1-159 | 5a-1 | A-27 | B-4 | 1-205 | 5a-2 | A-29 | B-1 | 1-251 | 5a-2 | A-31 | B-2 |
| 1-160 | 5a-1 | A-28 | B-4 | 1-206 | 5a-2 | A-30 | B-1 | 1-252 | 5a-2 | A-32 | B-2 |
| 1-161 | 5a-1 | A-29 | B-4 | 1-207 | 5a-2 | A-31 | B-1 | 1-253 | 5a-2 | A-33 | B-2 |
| 1-162 | 5a-1 | A-30 | B-4 | 1-208 | 5a-2 | A-32 | B-1 | 1-254 | 5a-2 | A-34 | B-2 |
| 1-163 | 5a-1 | A-31 | B-4 | 1-209 | 5a-2 | A-33 | B-1 | 1-255 | 5a-2 | A-35 | B-2 |
| 1-164 | 5a-1 | A-32 | B-4 | 1-210 | 5a-2 | A-34 | B-1 | 1-256 | 5a-2 | A-36 | B-2 |
| 1-165 | 5a-1 | A-33 | B-4 | 1-211 | 5a-2 | A-35 | B-1 | 1-257 | 5a-2 | A-37 | B-2 |
| 1-166 | 5a-1 | A-34 | B-4 | 1-212 | 5a-2 | A-36 | B-1 | 1-258 | 5a-2 | A-38 | B-2 |
| 1-167 | 5a-1 | A-35 | B-4 | 1-213 | 5a-2 | A-37 | B-1 | 1-259 | 5a-2 | A-39 | B-2 |
| 1-168 | 5a-1 | A-36 | B-4 | 1-214 | 5a-2 | A-38 | B-1 | 1-260 | 5a-2 | A-40 | B-2 |
| 1-169 | 5a-1 | A-37 | B-4 | 1-215 | 5a-2 | A-39 | B-1 | 1-261 | 5a-2 | A-41 | B-2 |
| 1-170 | 5a-1 | A-38 | B-4 | 1-216 | 5a-2 | A-40 | B-1 | 1-262 | 5a-2 | A-42 | B-2 |
| 1-171 | 5a-1 | A-39 | B-4 | 1-217 | 5a-2 | A-41 | B-1 | 1-263 | 5a-2 | A-43 | B-2 |
| 1-172 | 5a-1 | A-40 | B-4 | 1-218 | 5a-2 | A-42 | B-1 | 1-264 | 5a-2 | A-44 | B-2 |
| 1-173 | 5a-1 | A-41 | B-4 | 1-219 | 5a-2 | A-43 | B-1 | 1-265 | 5a-2 | A-1 | B-3 |
| 1-174 | 5a-1 | A-42 | B-4 | 1-220 | 5a-2 | A-44 | B-1 | 1-266 | 5a-2 | A-2 | B-3 |
| 1-175 | 5a-1 | A-43 | B-4 | 1-221 | 5a-2 | A-1 | B-2 | 1-267 | 5a-2 | A-3 | B-3 |
| 1-176 | 5a-1 | A-44 | B-4 | 1-222 | 5a-2 | A-2 | B-2 | 1-268 | 5a-2 | A-4 | B-3 |
| 1-177 | 5a-2 | A-1 | B-1 | 1-223 | 5a-2 | A-3 | B-2 | 1-269 | 5a-2 | A-5 | B-3 |
| 1-178 | 5a-2 | A-2 | B-1 | 1-224 | 5a-2 | A-4 | B-2 | 1-270 | 5a-2 | A-6 | B-3 |
| 1-179 | 5a-2 | A-3 | B-1 | 1-225 | 5a-2 | A-5 | B-2 | 1-271 | 5a-2 | A-7 | B-3 |
| 1-180 | 5a-2 | A-4 | B-1 | 1-226 | 5a-2 | A-6 | B-2 | 1-272 | 5a-2 | A-8 | B-3 |
| 1-181 | 5a-2 | A-5 | B-1 | 1-227 | 5a-2 | A-7 | B-2 | 1-273 | 5a-2 | A-9 | B-3 |
| 1-182 | 5a-2 | A-6 | B-1 | 1-228 | 5a-2 | A-8 | B-2 | 1-274 | 5a-2 | A-10 | B-3 |
| 1-183 | 5a-2 | A-7 | B-1 | 1-229 | 5a-2 | A-9 | B-2 | 1-275 | 5a-2 | A-11 | B-3 |
| 1-184 | 5a-2 | A-8 | B-1 | 1-230 | 5a-2 | A-10 | B-2 | 1-276 | 5a-2 | A-12 | B-3 |

**[Table 3]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-277 | 5a-2 | A-13 | B-3 | 1-323 | 5a-2 | A-15 | B-4 | 1-369 | 5b-1 | A-17 | B-1 |
| 1-278 | 5a-2 | A-14 | B-3 | 1-324 | 5a-2 | A-16 | B-4 | 1-370 | 5b-1 | A-18 | B-1 |
| 1-279 | 5a-2 | A-15 | B-3 | 1-325 | 5a-2 | A-17 | B-4 | 1-371 | 5b-1 | A-19 | B-1 |
| 1-280 | 5a-2 | A-16 | B-3 | 1-326 | 5a-2 | A-18 | B-4 | 1-372 | 5b-1 | A-20 | B-1 |
| 1-281 | 5a-2 | A-17 | B-3 | 1-327 | 5a-2 | A-19 | B-4 | 1-373 | 5b-1 | A-21 | B-1 |
| 1-282 | 5a-2 | A-18 | B-3 | 1-328 | 5a-2 | A-20 | B-4 | 1-374 | 5b-1 | A-22 | B-1 |
| 1-283 | 5a-2 | A-19 | B-3 | 1-329 | 5a-2 | A-21 | B-4 | 1-375 | 5b-1 | A-23 | B-1 |
| 1-284 | 5a-2 | A-20 | B-3 | 1-330 | 5a-2 | A-22 | B-4 | 1-376 | 5b-1 | A-24 | B-1 |
| 1-285 | 5a-2 | A-21 | B-3 | 1-331 | 5a-2 | A-23 | B-4 | 1-377 | 5b-1 | A-25 | B-1 |
| 1-286 | 5a-2 | A-22 | B-3 | 1-332 | 5a-2 | A-24 | B-4 | 1-378 | 5b-1 | A-26 | B-1 |
| 1-287 | 5a-2 | A-23 | B-3 | 1-333 | 5a-2 | A-25 | B-4 | 1-379 | 5b-1 | A-27 | B-1 |
| 1-288 | 5a-2 | A-24 | B-3 | 1-334 | 5a-2 | A-26 | B-4 | 1-380 | 5b-1 | A-28 | B-1 |
| 1-289 | 5a-2 | A-25 | B-3 | 1-335 | 5a-2 | A-27 | B-4 | 1-381 | 5b-1 | A-29 | B-1 |
| 1-290 | 5a-2 | A-26 | B-3 | 1-336 | 5a-2 | A-28 | B-4 | 1-382 | 5b-1 | A-30 | B-1 |
| 1-291 | 5a-2 | A-27 | B-3 | 1-337 | 5a-2 | A-29 | B-4 | 1-383 | 5b-1 | A-31 | B-1 |
| 1-292 | 5a-2 | A-28 | B-3 | 1-338 | 5a-2 | A-30 | B-4 | 1-384 | 5b-1 | A-32 | B-1 |
| 1-293 | 5a-2 | A-29 | B-3 | 1-339 | 5a-2 | A-31 | B-4 | 1-385 | 5b-1 | A-33 | B-1 |
| 1-294 | 5a-2 | A-30 | B-3 | 1-340 | 5a-2 | A-32 | B-4 | 1-386 | 5b-1 | A-34 | B-1 |
| 1-295 | 5a-2 | A-31 | B-3 | 1-341 | 5a-2 | A-33 | B-4 | 1-387 | 5b-1 | A-35 | B-1 |
| 1-296 | 5a-2 | A-32 | B-3 | 1-342 | 5a-2 | A-34 | B-4 | 1-388 | 5b-1 | A-36 | B-1 |
| 1-297 | 5a-2 | A-33 | B-3 | 1-343 | 5a-2 | A-35 | B-4 | 1-389 | 5b-1 | A-37 | B-1 |
| 1-298 | 5a-2 | A-34 | B-3 | 1-344 | 5a-2 | A-36 | B-4 | 1-390 | 5b-1 | A-38 | B-1 |
| 1-299 | 5a-2 | A-35 | B-3 | 1-345 | 5a-2 | A-37 | B-4 | 1-391 | 5b-1 | A-39 | B-1 |
| 1-300 | 5a-2 | A-36 | B-3 | 1-346 | 5a-2 | A-38 | B-4 | 1-392 | 5b-1 | A-40 | B-1 |
| 1-301 | 5a-2 | A-37 | B-3 | 1-347 | 5a-2 | A-39 | B-4 | 1-393 | 5b-1 | A-41 | B-1 |
| 1-302 | 5a-2 | A-38 | B-3 | 1-348 | 5a-2 | A-40 | B-4 | 1-394 | 5b-1 | A-42 | B-1 |
| 1-303 | 5a-2 | A-39 | B-3 | 1-349 | 5a-2 | A-41 | B-4 | 1-395 | 5b-1 | A-43 | B-1 |
| 1-304 | 5a-2 | A-40 | B-3 | 1-350 | 5a-2 | A-42 | B-4 | 1-396 | 5b-1 | A-44 | B-1 |
| 1-305 | 5a-2 | A-41 | B-3 | 1-351 | 5a-2 | A-43 | B-4 | 1-397 | 5b-1 | A-1 | B-2 |
| 1-306 | 5a-2 | A-42 | B-3 | 1-352 | 5a-2 | A-44 | B-4 | 1-398 | 5b-1 | A-2 | B-2 |
| 1-307 | 5a-2 | A-43 | B-3 | 1-353 | 5b-1 | A-1 | B-1 | 1-399 | 5b-1 | A-3 | B-2 |
| 1-308 | 5a-2 | A-44 | B-3 | 1-354 | 5b-1 | A-2 | B-1 | 1-400 | 5b-1 | A-4 | B-2 |
| 1-309 | 5a-2 | A-1 | B-4 | 1-355 | 5b-1 | A-3 | B-1 | 1-401 | 5b-1 | A-5 | B-2 |
| 1-310 | 5a-2 | A-2 | B-4 | 1-356 | 5b-1 | A-4 | B-1 | 1-402 | 5b-1 | A-6 | B-2 |
| 1-311 | 5a-2 | A-3 | B-4 | 1-357 | 5b-1 | A-5 | B-1 | 1-403 | 5b-1 | A-7 | B-2 |
| 1-312 | 5a-2 | A-4 | B-4 | 1-358 | 5b-1 | A-6 | B-1 | 1-404 | 5b-1 | A-8 | B-2 |
| 1-313 | 5a-2 | A-5 | B-4 | 1-359 | 5b-1 | A-7 | B-1 | 1-405 | 5b-1 | A-9 | B-2 |
| 1-314 | 5a-2 | A-6 | B-4 | 1-360 | 5b-1 | A-8 | B-1 | 1-406 | 5b-1 | A-10 | B-2 |
| 1-315 | 5a-2 | A-7 | B-4 | 1-361 | 5b-1 | A-9 | B-1 | 1-407 | 5b-1 | A-11 | B-2 |
| 1-316 | 5a-2 | A-8 | B-4 | 1-362 | 5b-1 | A-10 | B-1 | 1-408 | 5b-1 | A-12 | B-2 |
| 1-317 | 5a-2 | A-9 | B-4 | 1-363 | 5b-1 | A-11 | B-1 | 1-409 | 5b-1 | A-13 | B-2 |
| 1-318 | 5a-2 | A-10 | B-4 | 1-364 | 5b-1 | A-12 | B-1 | 1-410 | 5b-1 | A-14 | B-2 |
| 1-319 | 5a-2 | A-11 | B-4 | 1-365 | 5b-1 | A-13 | B-1 | 1-411 | 5b-1 | A-15 | B-2 |
| 1-320 | 5a-2 | A-12 | B-4 | 1-366 | 5b-1 | A-14 | B-1 | 1-412 | 5b-1 | A-16 | B-2 |
| 1-321 | 5a-2 | A-13 | B-4 | 1-367 | 5b-1 | A-15 | B-1 | 1-413 | 5b-1 | A-17 | B-2 |
| 1-322 | 5a-2 | A-14 | B-4 | 1-368 | 5b-1 | A-16 | B-1 | 1-414 | 5b-1 | A-18 | B-2 |

**[Table 4]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-415 | 5b-1 | A-19 | B-2 | 1-461 | 5b-1 | A-21 | B-3 | 1-507 | 5b-1 | A-23 | B-4 |
| 1-416 | 5b-1 | A-20 | B-2 | 1-462 | 5b-1 | A-22 | B-3 | 1-508 | 5b-1 | A-24 | B-4 |
| 1-417 | 5b-1 | A-21 | B-2 | 1-463 | 5b-1 | A-23 | B-3 | 1-509 | 5b-1 | A-25 | B-4 |
| 1-418 | 5b-1 | A-22 | B-2 | 1-464 | 5b-1 | A-24 | B-3 | 1-510 | 5b-1 | A-26 | B-4 |
| 1-419 | 5b-1 | A-23 | B-2 | 1-465 | 5b-1 | A-25 | B-3 | 1-511 | 5b-1 | A-27 | B-4 |
| 1-420 | 5b-1 | A-24 | B-2 | 1-466 | 5b-1 | A-26 | B-3 | 1-512 | 5b-1 | A-28 | B-4 |
| 1-421 | 5b-1 | A-25 | B-2 | 1-467 | 5b-1 | A-27 | B-3 | 1-513 | 5b-1 | A-29 | B-4 |
| 1-422 | 5b-1 | A-26 | B-2 | 1-468 | 5b-1 | A-28 | B-3 | 1-514 | 5b-1 | A-30 | B-4 |
| 1-423 | 5b-1 | A-27 | B-2 | 1-469 | 5b-1 | A-29 | B-3 | 1-515 | 5b-1 | A-31 | B-4 |
| 1-424 | 5b-1 | A-28 | B-2 | 1-470 | 5b-1 | A-30 | B-3 | 1-516 | 5b-1 | A-32 | B-4 |
| 1-425 | 5b-1 | A-29 | B-2 | 1-471 | 5b-1 | A-31 | B-3 | 1-517 | 5b-1 | A-33 | B-4 |
| 1-426 | 5b-1 | A-30 | B-2 | 1-472 | 5b-1 | A-32 | B-3 | 1-518 | 5b-1 | A-34 | B-4 |
| 1-427 | 5b-1 | A-31 | B-2 | 1-473 | 5b-1 | A-33 | B-3 | 1-519 | 5b-1 | A-35 | B-4 |
| 1-428 | 5b-1 | A-32 | B-2 | 1-474 | 5b-1 | A-34 | B-3 | 1-520 | 5b-1 | A-36 | B-4 |
| 1-429 | 5b-1 | A-33 | B-2 | 1-475 | 5b-1 | A-35 | B-3 | 1-521 | 5b-1 | A-37 | B-4 |
| 1-430 | 5b-1 | A-34 | B-2 | 1-476 | 5b-1 | A-36 | B-3 | 1-522 | 5b-1 | A-38 | B-4 |
| 1-431 | 5b-1 | A-35 | B-2 | 1-477 | 5b-1 | A-37 | B-3 | 1-523 | 5b-1 | A-39 | B-4 |
| 1-432 | 5b-1 | A-36 | B-2 | 1-478 | 5b-1 | A-38 | B-3 | 1-524 | 5b-1 | A-40 | B-4 |
| 1-433 | 5b-1 | A-37 | B-2 | 1-479 | 5b-1 | A-39 | B-3 | 1-525 | 5b-1 | A-41 | B-4 |
| 1-434 | 5b-1 | A-38 | B-2 | 1-480 | 5b-1 | A-40 | B-3 | 1-526 | 5b-1 | A-42 | B-4 |
| 1-435 | 5b-1 | A-39 | B-2 | 1-481 | 5b-1 | A-41 | B-3 | 1-527 | 5b-1 | A-43 | B-4 |
| 1-436 | 5b-1 | A-40 | B-2 | 1-482 | 5b-1 | A-42 | B-3 | 1-528 | 5b-1 | A-44 | B-4 |
| 1-437 | 5b-1 | A-41 | B-2 | 1-483 | 5b-1 | A-43 | B-3 | 1-529 | 5b-2 | A-1 | B-1 |
| 1-438 | 5b-1 | A-42 | B-2 | 1-484 | 5b-1 | A-44 | B-3 | 1-530 | 5b-2 | A-2 | B-1 |
| 1-439 | 5b-1 | A-43 | B-2 | 1-485 | 5b-1 | A-1 | B-4 | 1-531 | 5b-2 | A-3 | B-1 |
| 1-440 | 5b-1 | A-44 | B-2 | 1-486 | 5b-1 | A-2 | B-4 | 1-532 | 5b-2 | A-4 | B-1 |
| 1-441 | 5b-1 | A-1 | B-3 | 1-487 | 5b-1 | A-3 | B-4 | 1-533 | 5b-2 | A-5 | B-1 |
| 1-442 | 5b-1 | A-2 | B-3 | 1-488 | 5b-1 | A-4 | B-4 | 1-534 | 5b-2 | A-6 | B-1 |
| 1-443 | 5b-1 | A-3 | B-3 | 1-489 | 5b-1 | A-5 | B-4 | 1-535 | 5b-2 | A-7 | B-1 |
| 1-444 | 5b-1 | A-4 | B-3 | 1-490 | 5b-1 | A-6 | B-4 | 1-536 | 5b-2 | A-8 | B-1 |
| 1-445 | 5b-1 | A-5 | B-3 | 1-491 | 5b-1 | A-7 | B-4 | 1-537 | 5b-2 | A-9 | B-1 |
| 1-446 | 5b-1 | A-6 | B-3 | 1-492 | 5b-1 | A-8 | B-4 | 1-538 | 5b-2 | A-10 | B-1 |
| 1-447 | 5b-1 | A-7 | B-3 | 1-493 | 5b-1 | A-9 | B-4 | 1-539 | 5b-2 | A-11 | B-1 |
| 1-448 | 5b-1 | A-8 | B-3 | 1-494 | 5b-1 | A-10 | B-4 | 1-540 | 5b-2 | A-12 | B-1 |
| 1-449 | 5b-1 | A-9 | B-3 | 1-495 | 5b-1 | A-11 | B-4 | 1-541 | 5b-2 | A-13 | B-1 |
| 1-450 | 5b-1 | A-10 | B-3 | 1-496 | 5b-1 | A-12 | B-4 | 1-542 | 5b-2 | A-14 | B-1 |
| 1-451 | 5b-1 | A-11 | B-3 | 1-497 | 5b-1 | A-13 | B-4 | 1-543 | 5b-2 | A-15 | B-1 |
| 1-452 | 5b-1 | A-12 | B-3 | 1-498 | 5b-1 | A-14 | B-4 | 1-544 | 5b-2 | A-16 | B-1 |
| 1-453 | 5b-1 | A-13 | B-3 | 1-499 | 5b-1 | A-15 | B-4 | 1-545 | 5b-2 | A-17 | B-1 |
| 1-454 | 5b-1 | A-14 | B-3 | 1-500 | 5b-1 | A-16 | B-4 | 1-546 | 5b-2 | A-18 | B-1 |
| 1-455 | 5b-1 | A-15 | B-3 | 1-501 | 5b-1 | A-17 | B-4 | 1-547 | 5b-2 | A-19 | B-1 |
| 1-456 | 5b-1 | A-16 | B-3 | 1-502 | 5b-1 | A-18 | B-4 | 1-548 | 5b-2 | A-20 | B-1 |
| 1-457 | 5b-1 | A-17 | B-3 | 1-503 | 5b-1 | A-19 | B-4 | 1-549 | 5b-2 | A-21 | B-1 |
| 1-458 | 5b-1 | A-18 | B-3 | 1-504 | 5b-1 | A-20 | B-4 | 1-550 | 5b-2 | A-22 | B-1 |
| 1-459 | 5b-1 | A-19 | B-3 | 1-505 | 5b-1 | A-21 | B-4 | 1-551 | 5b-2 | A-23 | B-1 |
| 1-460 | 5b-1 | A-20 | B-3 | 1-506 | 5b-1 | A-22 | B-4 | 1-552 | 5b-2 | A-24 | B-1 |

**[Table 5]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-553 | 5b-2 | A-25 | B-1 | 1-599 | 5b-2 | A-27 | B-2 | 1-645 | 5b-2 | A-29 | B-3 |
| 1-554 | 5b-2 | A-26 | B-1 | 1-600 | 5b-2 | A-28 | B-2 | 1-646 | 5b-2 | A-30 | B-3 |
| 1-555 | 5b-2 | A-27 | B-1 | 1-601 | 5b-2 | A-29 | B-2 | 1-647 | 5b-2 | A-31 | B-3 |
| 1-556 | 5b-2 | A-28 | B-1 | 1-602 | 5b-2 | A-30 | B-2 | 1-648 | 5b-2 | A-32 | B-3 |
| 1-557 | 5b-2 | A-29 | B-1 | 1-603 | 5b-2 | A-31 | B-2 | 1-649 | 5b-2 | A-33 | B-3 |
| 1-558 | 5b-2 | A-30 | B-1 | 1-604 | 5b-2 | A-32 | B-2 | 1-650 | 5b-2 | A-34 | B-3 |
| 1-559 | 5b-2 | A-31 | B-1 | 1-605 | 5b-2 | A-33 | B-2 | 1-651 | 5b-2 | A-35 | B-3 |
| 1-560 | 5b-2 | A-32 | B-1 | 1-606 | 5b-2 | A-34 | B-2 | 1-652 | 5b-2 | A-36 | B-3 |
| 1-561 | 5b-2 | A-33 | B-1 | 1-607 | 5b-2 | A-35 | B-2 | 1-653 | 5b-2 | A-37 | B-3 |
| 1-562 | 5b-2 | A-34 | B-1 | 1-608 | 5b-2 | A-36 | B-2 | 1-654 | 5b-2 | A-38 | B-3 |
| 1-563 | 5b-2 | A-35 | B-1 | 1-609 | 5b-2 | A-37 | B-2 | 1-655 | 5b-2 | A-39 | B-3 |
| 1-564 | 5b-2 | A-36 | B-1 | 1-610 | 5b-2 | A-38 | B-2 | 1-656 | 5b-2 | A-40 | B-3 |
| 1-565 | 5b-2 | A-37 | B-1 | 1-611 | 5b-2 | A-39 | B-2 | 1-657 | 5b-2 | A-41 | B-3 |
| 1-566 | 5b-2 | A-38 | B-1 | 1-612 | 5b-2 | A-40 | B-2 | 1-658 | 5b-2 | A-42 | B-3 |
| 1-567 | 5b-2 | A-39 | B-1 | 1-613 | 5b-2 | A-41 | B-2 | 1-659 | 5b-2 | A-43 | B-3 |
| 1-568 | 5b-2 | A-40 | B-1 | 1-614 | 5b-2 | A-42 | B-2 | 1-660 | 5b-2 | A-44 | B-3 |
| 1-569 | 5b-2 | A-41 | B-1 | 1-615 | 5b-2 | A-43 | B-2 | 1-661 | 5b-2 | A-1 | B-4 |
| 1-570 | 5b-2 | A-42 | B-1 | 1-616 | 5b-2 | A-44 | B-2 | 1-662 | 5b-2 | A-2 | B-4 |
| 1-571 | 5b-2 | A-43 | B-1 | 1-617 | 5b-2 | A-1 | B-3 | 1-663 | 5b-2 | A-3 | B-4 |
| 1-572 | 5b-2 | A-44 | B-1 | 1-618 | 5b-2 | A-2 | B-3 | 1-664 | 5b-2 | A-4 | B-4 |
| 1-573 | 5b-2 | A-1 | B-2 | 1-619 | 5b-2 | A-3 | B-3 | 1-665 | 5b-2 | A-5 | B-4 |
| 1-574 | 5b-2 | A-2 | B-2 | 1-620 | 5b-2 | A-4 | B-3 | 1-666 | 5b-2 | A-6 | B-4 |
| 1-575 | 5b-2 | A-3 | B-2 | 1-621 | 5b-2 | A-5 | B-3 | 1-667 | 5b-2 | A-7 | B-4 |
| 1-576 | 5b-2 | A-4 | B-2 | 1-622 | 5b-2 | A-6 | B-3 | 1-668 | 5b-2 | A-8 | B-4 |
| 1-577 | 5b-2 | A-5 | B-2 | 1-623 | 5b-2 | A-7 | B-3 | 1-669 | 5b-2 | A-9 | B-4 |
| 1-578 | 5b-2 | A-6 | B-2 | 1-624 | 5b-2 | A-8 | B-3 | 1-670 | 5b-2 | A-10 | B-4 |
| 1-579 | 5b-2 | A-7 | B-2 | 1-625 | 5b-2 | A-9 | B-3 | 1-671 | 5b-2 | A-11 | B-4 |
| 1-580 | 5b-2 | A-8 | B-2 | 1-626 | 5b-2 | A-10 | B-3 | 1-672 | 5b-2 | A-12 | B-4 |
| 1-581 | 5b-2 | A-9 | B-2 | 1-627 | 5b-2 | A-11 | B-3 | 1-673 | 5b-2 | A-13 | B-4 |
| 1-582 | 5b-2 | A-10 | B-2 | 1-628 | 5b-2 | A-12 | B-3 | 1-674 | 5b-2 | A-14 | B-4 |
| 1-583 | 5b-2 | A-11 | B-2 | 1-629 | 5b-2 | A-13 | B-3 | 1-675 | 5b-2 | A-15 | B-4 |
| 1-584 | 5b-2 | A-12 | B-2 | 1-630 | 5b-2 | A-14 | B-3 | 1-676 | 5b-2 | A-16 | B-4 |
| 1-585 | 5b-2 | A-13 | B-2 | 1-631 | 5b-2 | A-15 | B-3 | 1-677 | 5b-2 | A-17 | B-4 |
| 1-586 | 5b-2 | A-14 | B-2 | 1-632 | 5b-2 | A-16 | B-3 | 1-678 | 5b-2 | A-18 | B-4 |
| 1-587 | 5b-2 | A-15 | B-2 | 1-633 | 5b-2 | A-17 | B-3 | 1-679 | 5b-2 | A-19 | B-4 |
| 1-588 | 5b-2 | A-16 | B-2 | 1-634 | 5b-2 | A-18 | B-3 | 1-680 | 5b-2 | A-20 | B-4 |
| 1-589 | 5b-2 | A-17 | B-2 | 1-635 | 5b-2 | A-19 | B-3 | 1-681 | 5b-2 | A-21 | B-4 |
| 1-590 | 5b-2 | A-18 | B-2 | 1-636 | 5b-2 | A-20 | B-3 | 1-682 | 5b-2 | A-22 | B-4 |
| 1-591 | 5b-2 | A-19 | B-2 | 1-637 | 5b-2 | A-21 | B-3 | 1-683 | 5b-2 | A-23 | B-4 |
| 1-592 | 5b-2 | A-20 | B-2 | 1-638 | 5b-2 | A-22 | B-3 | 1-684 | 5b-2 | A-24 | B-4 |
| 1-593 | 5b-2 | A-21 | B-2 | 1-639 | 5b-2 | A-23 | B-3 | 1-685 | 5b-2 | A-25 | B-4 |
| 1-594 | 5b-2 | A-22 | B-2 | 1-640 | 5b-2 | A-24 | B-3 | 1-686 | 5b-2 | A-26 | B-4 |
| 1-595 | 5b-2 | A-23 | B-2 | 1-641 | 5b-2 | A-25 | B-3 | 1-687 | 5b-2 | A-27 | B-4 |
| 1-596 | 5b-2 | A-24 | B-2 | 1-642 | 5b-2 | A-26 | B-3 | 1-688 | 5b-2 | A-28 | B-4 |
| 1-597 | 5b-2 | A-25 | B-2 | 1-643 | 5b-2 | A-27 | B-3 | 1-689 | 5b-2 | A-29 | B-4 |
| 1-598 | 5b-2 | A-26 | B-2 | 1-644 | 5b-2 | A-28 | B-3 | 1-690 | 5b-2 | A-30 | B-4 |

**[Table 6]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-691 | 5b-2 | A-31 | B-4 | 1-737 | 5c-1 | A-33 | B-1 | 1-783 | 5c-1 | A-35 | B-2 |
| 1-692 | 5b-2 | A-32 | B-4 | 1-738 | 5c-1 | A-34 | B-1 | 1-784 | 5c-1 | A-36 | B-2 |
| 1-693 | 5b-2 | A-33 | B-4 | 1-739 | 5c-1 | A-35 | B-1 | 1-785 | 5c-1 | A-37 | B-2 |
| 1-694 | 5b-2 | A-34 | B-4 | 1-740 | 5c-1 | A-36 | B-1 | 1-786 | 5c-1 | A-38 | B-2 |
| 1-695 | 5b-2 | A-35 | B-4 | 1-741 | 5c-1 | A-37 | B-1 | 1-787 | 5c-1 | A-39 | B-2 |
| 1-696 | 5b-2 | A-36 | B-4 | 1-742 | 5c-1 | A-38 | B-1 | 1-788 | 5c-1 | A-40 | B-2 |
| 1-697 | 5b-2 | A-37 | B-4 | 1-743 | 5c-1 | A-39 | B-1 | 1-789 | 5c-1 | A-41 | B-2 |
| 1-698 | 5b-2 | A-38 | B-4 | 1-744 | 5c-1 | A-40 | B-1 | 1-790 | 5c-1 | A-42 | B-2 |
| 1-699 | 5b-2 | A-39 | B-4 | 1-745 | 5c-1 | A-41 | B-1 | 1-791 | 5c-1 | A-43 | B-2 |
| 1-700 | 5b-2 | A-40 | B-4 | 1-746 | 5c-1 | A-42 | B-1 | 1-792 | 5c-1 | A-44 | B-2 |
| 1-701 | 5b-2 | A-41 | B-4 | 1-747 | 5c-1 | A-43 | B-1 | 1-793 | 5c-1 | A-1 | B-3 |
| 1-702 | 5b-2 | A-42 | B-4 | 1-748 | 5c-1 | A-44 | B-1 | 1-794 | 5c-1 | A-2 | B-3 |
| 1-703 | 5b-2 | A-43 | B-4 | 1-749 | 5c-1 | A-1 | B-2 | 1-795 | 5c-1 | A-3 | B-3 |
| 1-704 | 5b-2 | A-44 | B-4 | 1-750 | 5c-1 | A-2 | B-2 | 1-796 | 5c-1 | A-4 | B-3 |
| 1-705 | 5c-1 | A-1 | B-1 | 1-751 | 5c-1 | A-3 | B-2 | 1-797 | 5c-1 | A-5 | B-3 |
| 1-706 | 5c-1 | A-2 | B-1 | 1-752 | 5c-1 | A-4 | B-2 | 1-798 | 5c-1 | A-6 | B-3 |
| 1-707 | 5c-1 | A-3 | B-1 | 1-753 | 5c-1 | A-5 | B-2 | 1-799 | 5c-1 | A-7 | B-3 |
| 1-708 | 5c-1 | A-4 | B-1 | 1-754 | 5c-1 | A-6 | B-2 | 1-800 | 5c-1 | A-8 | B-3 |
| 1-709 | 5c-1 | A-5 | B-1 | 1-755 | 5c-1 | A-7 | B-2 | 1-801 | 5c-1 | A-9 | B-3 |
| 1-710 | 5c-1 | A-6 | B-1 | 1-756 | 5c-1 | A-8 | B-2 | 1-802 | 5c-1 | A-10 | B-3 |
| 1-711 | 5c-1 | A-7 | B-1 | 1-757 | 5c-1 | A-9 | B-2 | 1-803 | 5c-1 | A-11 | B-3 |
| 1-712 | 5c-1 | A-8 | B-1 | 1-758 | 5c-1 | A-10 | B-2 | 1-804 | 5c-1 | A-12 | B-3 |
| 1-713 | 5c-1 | A-9 | B-1 | 1-759 | 5c-1 | A-11 | B-2 | 1-805 | 5c-1 | A-13 | B-3 |
| 1-714 | 5c-1 | A-10 | B-1 | 1-760 | 5c-1 | A-12 | B-2 | 1-806 | 5c-1 | A-14 | B-3 |
| 1-715 | 5c-1 | A-11 | B-1 | 1-761 | 5c-1 | A-13 | B-2 | 1-807 | 5c-1 | A-15 | B-3 |
| 1-716 | 5c-1 | A-12 | B-1 | 1-762 | 5c-1 | A-14 | B-2 | 1-808 | 5c-1 | A-16 | B-3 |
| 1-717 | 5c-1 | A-13 | B-1 | 1-763 | 5c-1 | A-15 | B-2 | 1-809 | 5c-1 | A-17 | B-3 |
| 1-718 | 5c-1 | A-14 | B-1 | 1-764 | 5c-1 | A-16 | B-2 | 1-810 | 5c-1 | A-18 | B-3 |
| 1-719 | 5c-1 | A-15 | B-1 | 1-765 | 5c-1 | A-17 | B-2 | 1-811 | 5c-1 | A-19 | B-3 |
| 1-720 | 5c-1 | A-16 | B-1 | 1-766 | 5c-1 | A-18 | B-2 | 1-812 | 5c-1 | A-20 | B-3 |
| 1-721 | 5c-1 | A-17 | B-1 | 1-767 | 5c-1 | A-19 | B-2 | 1-813 | 5c-1 | A-21 | B-3 |
| 1-722 | 5c-1 | A-18 | B-1 | 1-768 | 5c-1 | A-20 | B-2 | 1-814 | 5c-1 | A-22 | B-3 |
| 1-723 | 5c-1 | A-19 | B-1 | 1-769 | 5c-1 | A-21 | B-2 | 1-815 | 5c-1 | A-23 | B-3 |
| 1-724 | 5c-1 | A-20 | B-1 | 1-770 | 5c-1 | A-22 | B-2 | 1-816 | 5c-1 | A-24 | B-3 |
| 1-725 | 5c-1 | A-21 | B-1 | 1-771 | 5c-1 | A-23 | B-2 | 1-817 | 5c-1 | A-25 | B-3 |
| 1-726 | 5c-1 | A-22 | B-1 | 1-772 | 5c-1 | A-24 | B-2 | 1-818 | 5c-1 | A-26 | B-3 |
| 1-727 | 5c-1 | A-23 | B-1 | 1-773 | 5c-1 | A-25 | B-2 | 1-819 | 5c-1 | A-27 | B-3 |
| 1-728 | 5c-1 | A-24 | B-1 | 1-774 | 5c-1 | A-26 | B-2 | 1-820 | 5c-1 | A-28 | B-3 |
| 1-729 | 5c-1 | A-25 | B-1 | 1-775 | 5c-1 | A-27 | B-2 | 1-821 | 5c-1 | A-29 | B-3 |
| 1-730 | 5c-1 | A-26 | B-1 | 1-776 | 5c-1 | A-28 | B-2 | 1-822 | 5c-1 | A-30 | B-3 |
| 1-731 | 5c-1 | A-27 | B-1 | 1-777 | 5c-1 | A-29 | B-2 | 1-823 | 5c-1 | A-31 | B-3 |
| 1-732 | 5c-1 | A-28 | B-1 | 1-778 | 5c-1 | A-30 | B-2 | 1-824 | 5c-1 | A-32 | B-3 |
| 1-733 | 5c-1 | A-29 | B-1 | 1-779 | 5c-1 | A-31 | B-2 | 1-825 | 5c-1 | A-33 | B-3 |
| 1-734 | 5c-1 | A-30 | B-1 | 1-780 | 5c-1 | A-32 | B-2 | 1-826 | 5c-1 | A-34 | B-3 |
| 1-735 | 5c-1 | A-31 | B-1 | 1-781 | 5c-1 | A-33 | B-2 | 1-827 | 5c-1 | A-35 | B-3 |
| 1-736 | 5c-1 | A-32 | B-1 | 1-782 | 5c-1 | A-34 | B-2 | 1-828 | 5c-1 | A-36 | B-3 |

**[Table 7]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-829 | 5c-1 | A-37 | B-3 | 1-875 | 5c-1 | A-39 | B-4 | 1-921 | 5c-2 | A-41 | B-1 |
| 1-830 | 5c-1 | A-38 | B-3 | 1-876 | 5c-1 | A-40 | B-4 | 1-922 | 5c-2 | A-42 | B-1 |
| 1-831 | 5c-1 | A-39 | B-3 | 1-877 | 5c-1 | A-41 | B-4 | 1-923 | 5c-2 | A-43 | B-1 |
| 1-832 | 5c-1 | A-40 | B-3 | 1-878 | 5c-1 | A-42 | B-4 | 1-924 | 5c-2 | A-44 | B-1 |
| 1-833 | 5c-1 | A-41 | B-3 | 1-879 | 5c-1 | A-43 | B-4 | 1-925 | 5c-2 | A-1 | B-2 |
| 1-834 | 5c-1 | A-42 | B-3 | 1-880 | 5c-1 | A-44 | B-4 | 1-926 | 5c-2 | A-2 | B-2 |
| 1-835 | 5c-1 | A-43 | B-3 | 1-881 | 5c-2 | A-1 | B-1 | 1-927 | 5c-2 | A-3 | B-2 |
| 1-836 | 5c-1 | A-44 | B-3 | 1-882 | 5c-2 | A-2 | B-1 | 1-928 | 5c-2 | A-4 | B-2 |
| 1-837 | 5c-1 | A-1 | B-4 | 1-883 | 5c-2 | A-3 | B-1 | 1-929 | 5c-2 | A-5 | B-2 |
| 1-838 | 5c-1 | A-2 | B-4 | 1-884 | 5c-2 | A-4 | B-1 | 1-930 | 5c-2 | A-6 | B-2 |
| 1-839 | 5c-1 | A-3 | B-4 | 1-885 | 5c-2 | A-5 | B-1 | 1-931 | 5c-2 | A-7 | B-2 |
| 1-840 | 5c-1 | A-4 | B-4 | 1-886 | 5c-2 | A-6 | B-1 | 1-932 | 5c-2 | A-8 | B-2 |
| 1-841 | 5c-1 | A-5 | B-4 | 1-887 | 5c-2 | A-7 | B-1 | 1-933 | 5c-2 | A-9 | B-2 |
| 1-842 | 5c-1 | A-6 | B-4 | 1-888 | 5c-2 | A-8 | B-1 | 1-934 | 5c-2 | A-10 | B-2 |
| 1-843 | 5c-1 | A-7 | B-4 | 1-889 | 5c-2 | A-9 | B-1 | 1-935 | 5c-2 | A-11 | B-2 |
| 1-844 | 5c-1 | A-8 | B-4 | 1-890 | 5c-2 | A-10 | B-1 | 1-936 | 5c-2 | A-12 | B-2 |
| 1-845 | 5c-1 | A-9 | B-4 | 1-891 | 5c-2 | A-11 | B-1 | 1-937 | 5c-2 | A-13 | B-2 |
| 1-846 | 5c-1 | A-10 | B-4 | 1-892 | 5c-2 | A-12 | B-1 | 1-938 | 5c-2 | A-14 | B-2 |
| 1-847 | 5c-1 | A-11 | B-4 | 1-893 | 5c-2 | A-13 | B-1 | 1-939 | 5c-2 | A-15 | B-2 |
| 1-848 | 5c-1 | A-12 | B-4 | 1-894 | 5c-2 | A-14 | B-1 | 1-940 | 5c-2 | A-16 | B-2 |
| 1-849 | 5c-1 | A-13 | B-4 | 1-895 | 5c-2 | A-15 | B-1 | 1-941 | 5c-2 | A-17 | B-2 |
| 1-850 | 5c-1 | A-14 | B-4 | 1-896 | 5c-2 | A-16 | B-1 | 1-942 | 5c-2 | A-18 | B-2 |
| 1-851 | 5c-1 | A-15 | B-4 | 1-897 | 5c-2 | A-17 | B-1 | 1-943 | 5c-2 | A-19 | B-2 |
| 1-852 | 5c-1 | A-16 | B-4 | 1-898 | 5c-2 | A-18 | B-1 | 1-944 | 5c-2 | A-20 | B-2 |
| 1-853 | 5c-1 | A-17 | B-4 | 1-899 | 5c-2 | A-19 | B-1 | 1-945 | 5c-2 | A-21 | B-2 |
| 1-854 | 5c-1 | A-18 | B-4 | 1-900 | 5c-2 | A-20 | B-1 | 1-946 | 5c-2 | A-22 | B-2 |
| 1-855 | 5c-1 | A-19 | B-4 | 1-901 | 5c-2 | A-21 | B-1 | 1-947 | 5c-2 | A-23 | B-2 |
| 1-856 | 5c-1 | A-20 | B-4 | 1-902 | 5c-2 | A-22 | B-1 | 1-948 | 5c-2 | A-24 | B-2 |
| 1-857 | 5c-1 | A-21 | B-4 | 1-903 | 5c-2 | A-23 | B-1 | 1-949 | 5c-2 | A-25 | B-2 |
| 1-858 | 5c-1 | A-22 | B-4 | 1-904 | 5c-2 | A-24 | B-1 | 1-950 | 5c-2 | A-26 | B-2 |
| 1-859 | 5c-1 | A-23 | B-4 | 1-905 | 5c-2 | A-25 | B-1 | 1-951 | 5c-2 | A-27 | B-2 |
| 1-860 | 5c-1 | A-24 | B-4 | 1-906 | 5c-2 | A-26 | B-1 | 1-952 | 5c-2 | A-28 | B-2 |
| 1-861 | 5c-1 | A-25 | B-4 | 1-907 | 5c-2 | A-27 | B-1 | 1-953 | 5c-2 | A-29 | B-2 |
| 1-862 | 5c-1 | A-26 | B-4 | 1-908 | 5c-2 | A-28 | B-1 | 1-954 | 5c-2 | A-30 | B-2 |
| 1-863 | 5c-1 | A-27 | B-4 | 1-909 | 5c-2 | A-29 | B-1 | 1-955 | 5c-2 | A-31 | B-2 |
| 1-864 | 5c-1 | A-28 | B-4 | 1-910 | 5c-2 | A-30 | B-1 | 1-956 | 5c-2 | A-32 | B-2 |
| 1-865 | 5c-1 | A-29 | B-4 | 1-911 | 5c-2 | A-31 | B-1 | 1-957 | 5c-2 | A-33 | B-2 |
| 1-866 | 5c-1 | A-30 | B-4 | 1-912 | 5c-2 | A-32 | B-1 | 1-958 | 5c-2 | A-34 | B-2 |
| 1-867 | 5c-1 | A-31 | B-4 | 1-913 | 5c-2 | A-33 | B-1 | 1-959 | 5c-2 | A-35 | B-2 |
| 1-868 | 5c-1 | A-32 | B-4 | 1-914 | 5c-2 | A-34 | B-1 | 1-960 | 5c-2 | A-36 | B-2 |
| 1-869 | 5c-1 | A-33 | B-4 | 1-915 | 5c-2 | A-35 | B-1 | 1-961 | 5c-2 | A-37 | B-2 |
| 1-870 | 5c-1 | A-34 | B-4 | 1-916 | 5c-2 | A-36 | B-1 | 1-962 | 5c-2 | A-38 | B-2 |
| 1-871 | 5c-1 | A-35 | B-4 | 1-917 | 5c-2 | A-37 | B-1 | 1-963 | 5c-2 | A-39 | B-2 |
| 1-872 | 5c-1 | A-36 | B-4 | 1-918 | 5c-2 | A-38 | B-1 | 1-964 | 5c-2 | A-40 | B-2 |
| 1-873 | 5c-1 | A-37 | B-4 | 1-919 | 5c-2 | A-39 | B-1 | 1-965 | 5c-2 | A-41 | B-2 |
| 1-874 | 5c-1 | A-38 | B-4 | 1-920 | 5c-2 | A-40 | B-1 | 1-966 | 5c-2 | A-42 | B-2 |

**[Table 8]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-967 | 5c-2 | A-43 | B-2 | 1-1013 | 5c-2 | A-1 | B-4 | 1-1059 | 5c-3 | A-3 | B-1 |
| 1-968 | 5c-2 | A-44 | B-2 | 1-1014 | 5c-2 | A-2 | B-4 | 1-1060 | 5c-3 | A-4 | B-1 |
| 1-969 | 5c-2 | A-1 | B-3 | 1-1015 | 5c-2 | A-3 | B-4 | 1-1061 | 5c-3 | A-5 | B-1 |
| 1-970 | 5c-2 | A-2 | B-3 | 1-1016 | 5c-2 | A-4 | B-4 | 1-1062 | 5c-3 | A-6 | B-1 |
| 1-971 | 5c-2 | A-3 | B-3 | 1-1017 | 5c-2 | A-5 | B-4 | 1-1063 | 5c-3 | A-7 | B-1 |
| 1-972 | 5c-2 | A-4 | B-3 | 1-1018 | 5c-2 | A-6 | B-4 | 1-1064 | 5c-3 | A-8 | B-1 |
| 1-973 | 5c-2 | A-5 | B-3 | 1-1019 | 5c-2 | A-7 | B-4 | 1-1065 | 5c-3 | A-9 | B-1 |
| 1-974 | 5c-2 | A-6 | B-3 | 1-1020 | 5c-2 | A-8 | B-4 | 1-1066 | 5c-3 | A-10 | B-1 |
| 1-975 | 5c-2 | A-7 | B-3 | 1-1021 | 5c-2 | A-9 | B-4 | 1-1067 | 5c-3 | A-11 | B-1 |
| 1-976 | 5c-2 | A-8 | B-3 | 1-1022 | 5c-2 | A-10 | B-4 | 1-1068 | 5c-3 | A-12 | B-1 |
| 1-977 | 5c-2 | A-9 | B-3 | 1-1023 | 5c-2 | A-11 | B-4 | 1-1069 | 5c-3 | A-13 | B-1 |
| 1-978 | 5c-2 | A-10 | B-3 | 1-1024 | 5c-2 | A-12 | B-4 | 1-1070 | 5c-3 | A-14 | B-1 |
| 1-979 | 5c-2 | A-11 | B-3 | 1-1025 | 5c-2 | A-13 | B-4 | 1-1071 | 5c-3 | A-15 | B-1 |
| 1-980 | 5c-2 | A-12 | B-3 | 1-1026 | 5c-2 | A-14 | B-4 | 1-1072 | 5c-3 | A-16 | B-1 |
| 1-981 | 5c-2 | A-13 | B-3 | 1-1027 | 5c-2 | A-15 | B-4 | 1-1073 | 5c-3 | A-17 | B-1 |
| 1-982 | 5c-2 | A-14 | B-3 | 1-1028 | 5c-2 | A-16 | B-4 | 1-1074 | 5c-3 | A-18 | B-1 |
| 1-983 | 5c-2 | A-15 | B-3 | 1-1029 | 5c-2 | A-17 | B-4 | 1-1075 | 5c-3 | A-19 | B-1 |
| 1-984 | 5c-2 | A-16 | B-3 | 1-1030 | 5c-2 | A-18 | B-4 | 1-1076 | 5c-3 | A-20 | B-1 |
| 1-985 | 5c-2 | A-17 | B-3 | 1-1031 | 5c-2 | A-19 | B-4 | 1-1077 | 5c-3 | A-21 | B-1 |
| 1-986 | 5c-2 | A-18 | B-3 | 1-1032 | 5c-2 | A-20 | B-4 | 1-1078 | 5c-3 | A-22 | B-1 |
| 1-987 | 5c-2 | A-19 | B-3 | 1-1033 | 5c-2 | A-21 | B-4 | 1-1079 | 5c-3 | A-23 | B-1 |
| 1-988 | 5c-2 | A-20 | B-3 | 1-1034 | 5c-2 | A-22 | B-4 | 1-1080 | 5c-3 | A-24 | B-1 |
| 1-989 | 5c-2 | A-21 | B-3 | 1-1035 | 5c-2 | A-23 | B-4 | 1-1081 | 5c-3 | A-25 | B-1 |
| 1-990 | 5c-2 | A-22 | B-3 | 1-1036 | 5c-2 | A-24 | B-4 | 1-1082 | 5c-3 | A-26 | B-1 |
| 1-991 | 5c-2 | A-23 | B-3 | 1-1037 | 5c-2 | A-25 | B-4 | 1-1083 | 5c-3 | A-27 | B-1 |
| 1-992 | 5c-2 | A-24 | B-3 | 1-1038 | 5c-2 | A-26 | B-4 | 1-1084 | 5c-3 | A-28 | B-1 |
| 1-993 | 5c-2 | A-25 | B-3 | 1-1039 | 5c-2 | A-27 | B-4 | 1-1085 | 5c-3 | A-29 | B-1 |
| 1-994 | 5c-2 | A-26 | B-3 | 1-1040 | 5c-2 | A-28 | B-4 | 1-1086 | 5c-3 | A-30 | B-1 |
| 1-995 | 5c-2 | A-27 | B-3 | 1-1041 | 5c-2 | A-29 | B-4 | 1-1087 | 5c-3 | A-31 | B-1 |
| 1-996 | 5c-2 | A-28 | B-3 | 1-1042 | 5c-2 | A-30 | B-4 | 1-1088 | 5c-3 | A-32 | B-1 |
| 1-997 | 5c-2 | A-29 | B-3 | 1-1043 | 5c-2 | A-31 | B-4 | 1-1089 | 5c-3 | A-33 | B-1 |
| 1-998 | 5c-2 | A-30 | B-3 | 1-1044 | 5c-2 | A-32 | B-4 | 1-1090 | 5c-3 | A-34 | B-1 |
| 1-999 | 5c-2 | A-31 | B-3 | 1-1045 | 5c-2 | A-33 | B-4 | 1-1091 | 5c-3 | A-35 | B-1 |
| 1-1000 | 5c-2 | A-32 | B-3 | 1-1046 | 5c-2 | A-34 | B-4 | 1-1092 | 5c-3 | A-36 | B-1 |
| 1-1001 | 5c-2 | A-33 | B-3 | 1-1047 | 5c-2 | A-35 | B-4 | 1-1093 | 5c-3 | A-37 | B-1 |
| 1-1002 | 5c-2 | A-34 | B-3 | 1-1048 | 5c-2 | A-36 | B-4 | 1-1094 | 5c-3 | A-38 | B-1 |
| 1-1003 | 5c-2 | A-35 | B-3 | 1-1049 | 5c-2 | A-37 | B-4 | 1-1095 | 5c-3 | A-39 | B-1 |
| 1-1004 | 5c-2 | A-36 | B-3 | 1-1050 | 5c-2 | A-38 | B-4 | 1-1096 | 5c-3 | A-40 | B-1 |
| 1-1005 | 5c-2 | A-37 | B-3 | 1-1051 | 5c-2 | A-39 | B-4 | 1-1097 | 5c-3 | A-41 | B-1 |
| 1-1006 | 5c-2 | A-38 | B-3 | 1-1052 | 5c-2 | A-40 | B-4 | 1-1098 | 5c-3 | A-42 | B-1 |
| 1-1007 | 5c-2 | A-39 | B-3 | 1-1053 | 5c-2 | A-41 | B-4 | 1-1099 | 5c-3 | A-43 | B-1 |
| 1-1008 | 5c-2 | A-40 | B-3 | 1-1054 | 5c-2 | A-42 | B-4 | 1-1100 | 5c-3 | A-44 | B-1 |
| 1-1009 | 5c-2 | A-41 | B-3 | 1-1055 | 5c-2 | A-43 | B-4 | 1-1101 | 5c-3 | A-1 | B-2 |
| 1-1010 | 5c-2 | A-42 | B-3 | 1-1056 | 5c-2 | A-44 | B-4 | 1-1102 | 5c-3 | A-2 | B-2 |
| 1-1011 | 5c-2 | A-43 | B-3 | 1-1057 | 5c-3 | A-1 | B-1 | 1-1103 | 5c-3 | A-3 | B-2 |
| 1-1012 | 5c-2 | A-44 | B-3 | 1-1058 | 5c-3 | A-2 | B-1 | 1-1104 | 5c-3 | A-4 | B-2 |

**[Table 9]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-1105 | 5c-3 | A-5 | B-2 | 1-1151 | 5c-3 | A-7 | B-3 | 1-1197 | 5c-3 | A-9 | B-4 |
| 1-1106 | 5c-3 | A-6 | B-2 | 1-1152 | 5c-3 | A-8 | B-3 | 1-1198 | 5c-3 | A-10 | B-4 |
| 1-1107 | 5c-3 | A-7 | B-2 | 1-1153 | 5c-3 | A-9 | B-3 | 1-1199 | 5c-3 | A-11 | B-4 |
| 1-1108 | 5c-3 | A-8 | B-2 | 1-1154 | 5c-3 | A-10 | B-3 | 1-1200 | 5c-3 | A-12 | B-4 |
| 1-1109 | 5c-3 | A-9 | B-2 | 1-1155 | 5c-3 | A-11 | B-3 | 1-1201 | 5c-3 | A-13 | B-4 |
| 1-1110 | 5c-3 | A-10 | B-2 | 1-1156 | 5c-3 | A-12 | B-3 | 1-1202 | 5c-3 | A-14 | B-4 |
| 1-1111 | 5c-3 | A-11 | B-2 | 1-1157 | 5c-3 | A-13 | B-3 | 1-1203 | 5c-3 | A-15 | B-4 |
| 1-1112 | 5c-3 | A-12 | B-2 | 1-1158 | 5c-3 | A-14 | B-3 | 1-1204 | 5c-3 | A-16 | B-4 |
| 1-1113 | 5c-3 | A-13 | B-2 | 1-1159 | 5c-3 | A-15 | B-3 | 1-1205 | 5c-3 | A-17 | B-4 |
| 1-1114 | 5c-3 | A-14 | B-2 | 1-1160 | 5c-3 | A-16 | B-3 | 1-1206 | 5c-3 | A-18 | B-4 |
| 1-1115 | 5c-3 | A-15 | B-2 | 1-1161 | 5c-3 | A-17 | B-3 | 1-1207 | 5c-3 | A-19 | B-4 |
| 1-1116 | 5c-3 | A-16 | B-2 | 1-1162 | 5c-3 | A-18 | B-3 | 1-1208 | 5c-3 | A-20 | B-4 |
| 1-1117 | 5c-3 | A-17 | B-2 | 1-1163 | 5c-3 | A-19 | B-3 | 1-1209 | 5c-3 | A-21 | B-4 |
| 1-1118 | 5c-3 | A-18 | B-2 | 1-1164 | 5c-3 | A-20 | B-3 | 1-1210 | 5c-3 | A-22 | B-4 |
| 1-1119 | 5c-3 | A-19 | B-2 | 1-1165 | 5c-3 | A-21 | B-3 | 1-1211 | 5c-3 | A-23 | B-4 |
| 1-1120 | 5c-3 | A-20 | B-2 | 1-1166 | 5c-3 | A-22 | B-3 | 1-1212 | 5c-3 | A-24 | B-4 |
| 1-1121 | 5c-3 | A-21 | B-2 | 1-1167 | 5c-3 | A-23 | B-3 | 1-1213 | 5c-3 | A-25 | B-4 |
| 1-1122 | 5c-3 | A-22 | B-2 | 1-1168 | 5c-3 | A-24 | B-3 | 1-1214 | 5c-3 | A-26 | B-4 |
| 1-1123 | 5c-3 | A-23 | B-2 | 1-1169 | 5c-3 | A-25 | B-3 | 1-1215 | 5c-3 | A-27 | B-4 |
| 1-1124 | 5c-3 | A-24 | B-2 | 1-1170 | 5c-3 | A-26 | B-3 | 1-1216 | 5c-3 | A-28 | B-4 |
| 1-1125 | 5c-3 | A-25 | B-2 | 1-1171 | 5c-3 | A-27 | B-3 | 1-1217 | 5c-3 | A-29 | B-4 |
| 1-1126 | 5c-3 | A-26 | B-2 | 1-1172 | 5c-3 | A-28 | B-3 | 1-1218 | 5c-3 | A-30 | B-4 |
| 1-1127 | 5c-3 | A-27 | B-2 | 1-1173 | 5c-3 | A-29 | B-3 | 1-1219 | 5c-3 | A-31 | B-4 |
| 1-1128 | 5c-3 | A-28 | B-2 | 1-1174 | 5c-3 | A-30 | B-3 | 1-1220 | 5c-3 | A-32 | B-4 |
| 1-1129 | 5c-3 | A-29 | B-2 | 1-1175 | 5c-3 | A-31 | B-3 | 1-1221 | 5c-3 | A-33 | B-4 |
| 1-1130 | 5c-3 | A-30 | B-2 | 1-1176 | 5c-3 | A-32 | B-3 | 1-1222 | 5c-3 | A-34 | B-4 |
| 1-1131 | 5c-3 | A-31 | B-2 | 1-1177 | 5c-3 | A-33 | B-3 | 1-1223 | 5c-3 | A-35 | B-4 |
| 1-1132 | 5c-3 | A-32 | B-2 | 1-1178 | 5c-3 | A-34 | B-3 | 1-1224 | 5c-3 | A-36 | B-4 |
| 1-1133 | 5c-3 | A-33 | B-2 | 1-1179 | 5c-3 | A-35 | B-3 | 1-1225 | 5c-3 | A-37 | B-4 |
| 1-1134 | 5c-3 | A-34 | B-2 | 1-1180 | 5c-3 | A-36 | B-3 | 1-1226 | 5c-3 | A-38 | B-4 |
| 1-1135 | 5c-3 | A-35 | B-2 | 1-1181 | 5c-3 | A-37 | B-3 | 1-1227 | 5c-3 | A-39 | B-4 |
| 1-1136 | 5c-3 | A-36 | B-2 | 1-1182 | 5c-3 | A-38 | B-3 | 1-1228 | 5c-3 | A-40 | B-4 |
| 1-1137 | 5c-3 | A-37 | B-2 | 1-1183 | 5c-3 | A-39 | B-3 | 1-1229 | 5c-3 | A-41 | B-4 |
| 1-1138 | 5c-3 | A-38 | B-2 | 1-1184 | 5c-3 | A-40 | B-3 | 1-1230 | 5c-3 | A-42 | B-4 |
| 1-1139 | 5c-3 | A-39 | B-2 | 1-1185 | 5c-3 | A-41 | B-3 | 1-1231 | 5c-3 | A-43 | B-4 |
| 1-1140 | 5c-3 | A-40 | B-2 | 1-1186 | 5c-3 | A-42 | B-3 | 1-1232 | 5c-3 | A-44 | B-4 |
| 1-1141 | 5c-3 | A-41 | B-2 | 1-1187 | 5c-3 | A-43 | B-3 | 1-1233 | 5c-4 | A-1 | B-1 |
| 1-1142 | 5c-3 | A-42 | B-2 | 1-1188 | 5c-3 | A-44 | B-3 | 1-1234 | 5c-4 | A-2 | B-1 |
| 1-1143 | 5c-3 | A-43 | B-2 | 1-1189 | 5c-3 | A-1 | B-4 | 1-1235 | 5c-4 | A-3 | B-1 |
| 1-1144 | 5c-3 | A-44 | B-2 | 1-1190 | 5c-3 | A-2 | B-4 | 1-1236 | 5c-4 | A-4 | B-1 |
| 1-1145 | 5c-3 | A-1 | B-3 | 1-1191 | 5c-3 | A-3 | B-4 | 1-1237 | 5c-4 | A-5 | B-1 |
| 1-1146 | 5c-3 | A-2 | B-3 | 1-1192 | 5c-3 | A-4 | B-4 | 1-1238 | 5c-4 | A-6 | B-1 |
| 1-1147 | 5c-3 | A-3 | B-3 | 1-1193 | 5c-3 | A-5 | B-4 | 1-1239 | 5c-4 | A-7 | B-1 |
| 1-1148 | 5c-3 | A-4 | B-3 | 1-1194 | 5c-3 | A-6 | B-4 | 1-1240 | 5c-4 | A-8 | B-1 |
| 1-1149 | 5c-3 | A-5 | B-3 | 1-1195 | 5c-3 | A-7 | B-4 | 1-1241 | 5c-4 | A-9 | B-1 |
| 1-1150 | 5c-3 | A-6 | B-3 | 1-1196 | 5c-3 | A-8 | B-4 | 1-1242 | 5c-4 | A-10 | B-1 |

**[Table 10]**

| Compound No. | Group 4 | X¹ | X² | Compound No. | Group 4 | X¹ | X² | Compound No. | Group 4 | X¹ | X² |
|---|---|---|---|---|---|---|---|---|---|---|---|
| 1-1243 | 5c-4 | A-11 | B-1 | 1-1289 | 5c-4 | A-13 | B-2 | 1-1335 | 5c-4 | A-15 | B-3 |
| 1-1244 | 5c-4 | A-12 | B-1 | 1-1290 | 5c-4 | A-14 | B-2 | 1-1336 | 5c-4 | A-16 | B-3 |
| 1-1245 | 5c-4 | A-13 | B-1 | 1-1291 | 5c-4 | A-15 | B-2 | 1-1337 | 5c-4 | A-17 | B-3 |
| 1-1246 | 5c-4 | A-14 | B-1 | 1-1292 | 5c-4 | A-16 | B-2 | 1-1338 | 5c-4 | A-18 | B-3 |
| 1-1247 | 5c-4 | A-15 | B-1 | 1-1293 | 5c-4 | A-17 | B-2 | 1-1339 | 5c-4 | A-19 | B-3 |
| 1-1248 | 5c-4 | A-16 | B-1 | 1-1294 | 5c-4 | A-18 | B-2 | 1-1340 | 5c-4 | A-20 | B-3 |
| 1-1249 | 5c-4 | A-17 | B-1 | 1-1295 | 5c-4 | A-19 | B-2 | 1-1341 | 5c-4 | A-21 | B-3 |
| 1-1250 | 5c-4 | A-18 | B-1 | 1-1296 | 5c-4 | A-20 | B-2 | 1-1342 | 5c-4 | A-22 | B-3 |
| 1-1251 | 5c-4 | A-19 | B-1 | 1-1297 | 5c-4 | A-21 | B-2 | 1-1343 | 5c-4 | A-23 | B-3 |
| 1-1252 | 5c-4 | A-20 | B-1 | 1-1298 | 5c-4 | A-22 | B-2 | 1-1344 | 5c-4 | A-24 | B-3 |
| 1-1253 | 5c-4 | A-21 | B-1 | 1-1299 | 5c-4 | A-23 | B-2 | 1-1345 | 5c-4 | A-25 | B-3 |
| 1-1254 | 5c-4 | A-22 | B-1 | 1-1300 | 5c-4 | A-24 | B-2 | 1-1346 | 5c-4 | A-26 | B-3 |
| 1-1255 | 5c-4 | A-23 | B-1 | 1-1301 | 5c-4 | A-25 | B-2 | 1-1347 | 5c-4 | A-27 | B-3 |
| 1-1256 | 5c-4 | A-24 | B-1 | 1-1302 | 5c-4 | A-26 | B-2 | 1-1348 | 5c-4 | A-28 | B-3 |
| 1-1257 | 5c-4 | A-25 | B-1 | 1-1303 | 5c-4 | A-27 | B-2 | 1-1349 | 5c-4 | A-29 | B-3 |
| 1-1258 | 5c-4 | A-26 | B-1 | 1-1304 | 5c-4 | A-28 | B-2 | 1-1350 | 5c-4 | A-30 | B-3 |
| 1-1259 | 5c-4 | A-27 | B-1 | 1-1305 | 5c-4 | A-29 | B-2 | 1-1351 | 5c-4 | A-31 | B-3 |
| 1-1260 | 5c-4 | A-28 | B-1 | 1-1306 | 5c-4 | A-30 | B-2 | 1-1352 | 5c-4 | A-32 | B-3 |
| 1-1261 | 5c-4 | A-29 | B-1 | 1-1307 | 5c-4 | A-31 | B-2 | 1-1353 | 5c-4 | A-33 | B-3 |
| 1-1262 | 5c-4 | A-30 | B-1 | 1-1308 | 5c-4 | A-32 | B-2 | 1-1354 | 5c-4 | A-34 | B-3 |
| 1-1263 | 5c-4 | A-31 | B-1 | 1-1309 | 5c-4 | A-33 | B-2 | 1-1355 | 5c-4 | A-35 | B-3 |
| 1-1264 | 5c-4 | A-32 | B-1 | 1-1310 | 5c-4 | A-34 | B-2 | 1-1356 | 5c-4 | A-36 | B-3 |
| 1-1265 | 5c-4 | A-33 | B-1 | 1-1311 | 5c-4 | A-35 | B-2 | 1-1357 | 5c-4 | A-37 | B-3 |
| 1-1266 | 5c-4 | A-34 | B-1 | 1-1312 | 5c-4 | A-36 | B-2 | 1-1358 | 5c-4 | A-38 | B-3 |
| 1-1267 | 5c-4 | A-35 | B-1 | 1-1313 | 5c-4 | A-37 | B-2 | 1-1359 | 5c-4 | A-39 | B-3 |
| 1-1268 | 5c-4 | A-36 | B-1 | 1-1314 | 5c-4 | A-38 | B-2 | 1-1360 | 5c-4 | A-40 | B-3 |
| 1-1269 | 5c-4 | A-37 | B-1 | 1-1315 | 5c-4 | A-39 | B-2 | 1-1361 | 5c-4 | A-41 | B-3 |
| 1-1270 | 5c-4 | A-38 | B-1 | 1-1316 | 5c-4 | A-40 | B-2 | 1-1362 | 5c-4 | A-42 | B-3 |
| 1-1271 | 5c-4 | A-39 | B-1 | 1-1317 | 5c-4 | A-41 | B-2 | 1-1363 | 5c-4 | A-43 | B-3 |
| 1-1272 | 5c-4 | A-40 | B-1 | 1-1318 | 5c-4 | A-42 | B-2 | 1-1364 | 5c-4 | A-44 | B-3 |
| 1-1273 | 5c-4 | A-41 | B-1 | 1-1319 | 5c-4 | A-43 | B-2 | 1-1365 | 5c-4 | A-1 | B-4 |
| 1-1274 | 5c-4 | A-42 | B-1 | 1-1320 | 5c-4 | A-44 | B-2 | 1-1366 | 5c-4 | A-2 | B-4 |
| 1-1275 | 5c-4 | A-43 | B-1 | 1-1321 | 5c-4 | A-1 | B-3 | 1-1367 | 5c-4 | A-3 | B-4 |
| 1-1276 | 5c-4 | A-44 | B-1 | 1-1322 | 5c-4 | A-2 | B-3 | 1-1368 | 5c-4 | A-4 | B-4 |
| 1-1277 | 5c-4 | A-1 | B-2 | 1-1323 | 5c-4 | A-3 | B-3 | 1-1369 | 5c-4 | A-5 | B-4 |
| 1-1278 | 5c-4 | A-2 | B-2 | 1-1324 | 5c-4 | A-4 | B-3 | 1-1370 | 5c-4 | A-6 | B-4 |
| 1-1279 | 5c-4 | A-3 | B-2 | 1-1325 | 5c-4 | A-5 | B-3 | 1-1371 | 5c-4 | A-7 | B-4 |
| 1-1280 | 5c-4 | A-4 | B-2 | 1-1326 | 5c-4 | A-6 | B-3 | 1-1372 | 5c-4 | A-8 | B-4 |
| 1-1281 | 5c-4 | A-5 | B-2 | 1-1327 | 5c-4 | A-7 | B-3 | 1-1373 | 5c-4 | A-9 | B-4 |
| 1-1282 | 5c-4 | A-6 | B-2 | 1-1328 | 5c-4 | A-8 | B-3 | 1-1374 | 5c-4 | A-10 | B-4 |
| 1-1283 | 5c-4 | A-7 | B-2 | 1-1329 | 5c-4 | A-9 | B-3 | 1-1375 | 5c-4 | A-11 | B-4 |
| 1-1284 | 5c-4 | A-8 | B-2 | 1-1330 | 5c-4 | A-10 | B-3 | 1-1376 | 5c-4 | A-12 | B-4 |
| 1-1285 | 5c-4 | A-9 | B-2 | 1-1331 | 5c-4 | A-11 | B-3 | 1-1377 | 5c-4 | A-13 | B-4 |
| 1-1286 | 5c-4 | A-10 | B-2 | 1-1332 | 5c-4 | A-12 | B-3 | 1-1378 | 5c-4 | A-14 | B-4 |
| 1-1287 | 5c-4 | A-11 | B-2 | 1-1333 | 5c-4 | A-13 | B-3 | 1-1379 | 5c-4 | A-15 | B-4 |
| 1-1288 | 5c-4 | A-12 | B-2 | 1-1334 | 5c-4 | A-14 | B-3 | 1-1380 | 5c-4 | A-16 | B-4 |

**[Table 11]**

| Compound No. | Group 4 | X¹ | X² |
|---|---|---|---|
| 1-1381 | 5c-4 | A-17 | B-4 |
| 1-1382 | 5c-4 | A-18 | B-4 |
| 1-1383 | 5c-4 | A-19 | B-4 |
| 1-1384 | 5c-4 | A-20 | B-4 |
| 1-1385 | 5c-4 | A-21 | B-4 |
| 1-1386 | 5c-4 | A-22 | B-4 |
| 1-1387 | 5c-4 | A-23 | B-4 |
| 1-1388 | 5c-4 | A-24 | B-4 |
| 1-1389 | 5c-4 | A-25 | B-4 |
| 1-1390 | 5c-4 | A-26 | B-4 |
| 1-1391 | 5c-4 | A-27 | B-4 |
| 1-1392 | 5c-4 | A-28 | B-4 |
| 1-1393 | 5c-4 | A-29 | B-4 |
| 1-1394 | 5c-4 | A-30 | B-4 |
| 1-1395 | 5c-4 | A-31 | B-4 |
| 1-1396 | 5c-4 | A-32 | B-4 |
| 1-1397 | 5c-4 | A-33 | B-4 |
| 1-1398 | 5c-4 | A-34 | B-4 |
| 1-1399 | 5c-4 | A-35 | B-4 |
| 1-1400 | 5c-4 | A-36 | B-4 |
| 1-1401 | 5c-4 | A-37 | B-4 |
| 1-1402 | 5c-4 | A-38 | B-4 |
| 1-1403 | 5c-4 | A-39 | B-4 |
| 1-1404 | 5c-4 | A-40 | B-4 |
| 1-1405 | 5c-4 | A-41 | B-4 |
| 1-1406 | 5c-4 | A-42 | B-4 |
| 1-1407 | 5c-4 | A-43 | B-4 |
| 1-1408 | 5c-4 | A-44 | B-4 |

The second host compound may include at least one carbazole group with a substituent having hole characteristics.

The second host compound is, for example represented by the Chemical Formula 5.

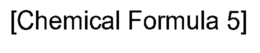

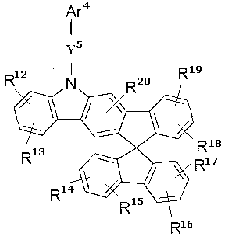

In the Chemical Formula 5,
Ar⁴ is a substituted or unsubstituted C6 to C30 aryl group, a substituted or unsubstituted C2 to C30 heteroaryl group, or a combination thereof,
Y⁵ is a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof, and
R¹² to R²⁰ are independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

In the Chemical Formula 5, Ar⁴ may be a substituent having hole characteristics, for example a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted carbazolyl group, a substituted or unsubstituted benzofuranyl group, a substituted or unsubstituted benzothiophenyl group, a substituted or unsubstituted fluorenyl group, a substituted or unsubstituted dibenzothiophenyl group, a substituted or unsubstituted dibenzofuranyl group, or a combination thereof.

The second host compound represented by the Chemical Formula 5 may be, for example one of compounds listed in the following Group 6, but is not limited thereto.

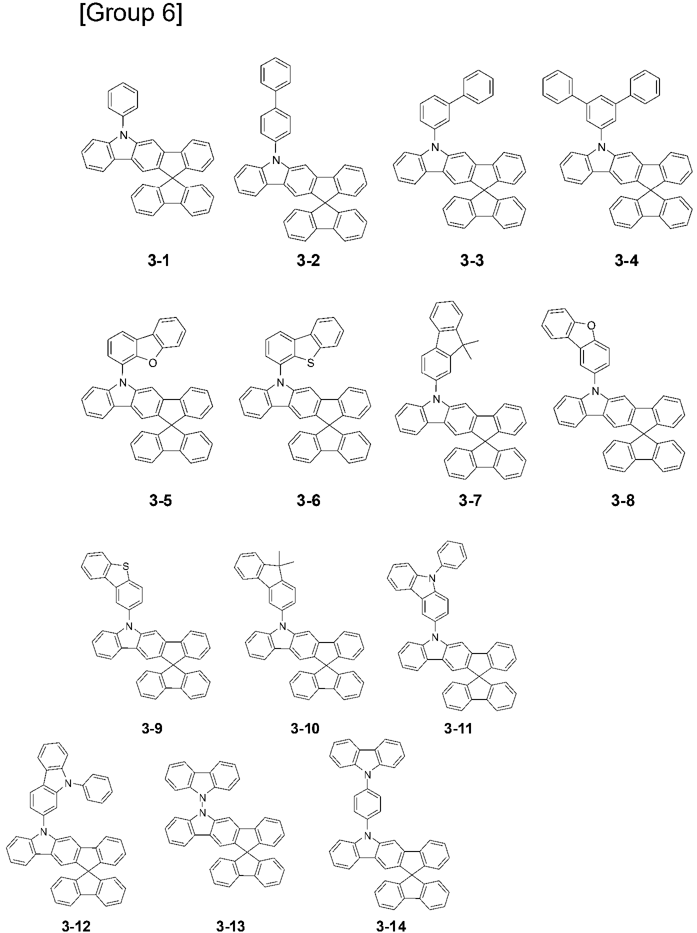

The second host compound is, for example represented by the Chemical Formula 6. Chemical Formula 7 only represents background art.

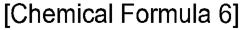

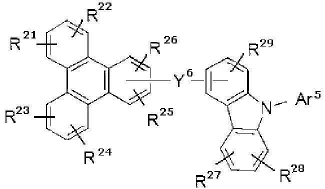

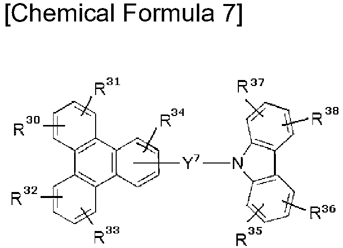

In the Chemical Formula 6,
Ar⁵ is a substituted or unsubstituted C6 to C30 aryl group, a substituted or unsubstituted C2 to C30 heteroaryl group, or a combination thereof,
Y⁶ and Y⁷ are independently a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof, and
R²¹ to R³⁸ are independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

In the Chemical Formula 6, Ar⁵ may be a substituent having hole characteristics, for example a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted carbazolyl group, a substituted or unsubstituted benzofuranyl group, a substituted or unsubstituted benzothiophenyl group, a substituted or unsubstituted fluorenyl group, a substituted or unsubstituted dibenzothiophenyl group, a substituted or unsubstituted dibenzofuranyl group, or a combination thereof.

The second host compound represented by the Chemical Formula 6 may be, for example one of compounds listed in the following Group 7, but is not limited thereto.

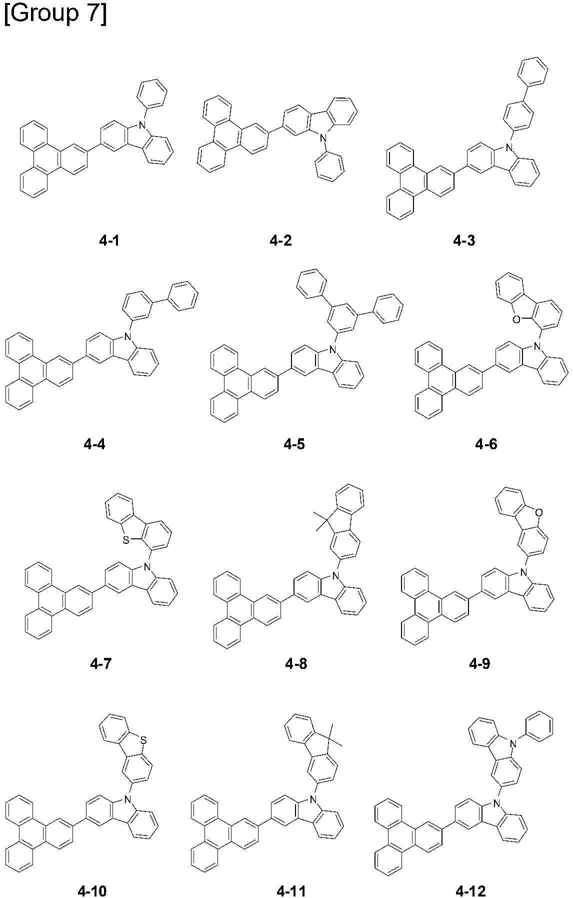

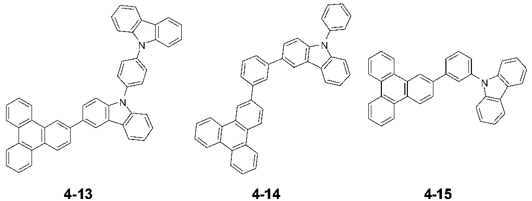

Compounds 4-15 and 4-17 to 4-19 represent background art.

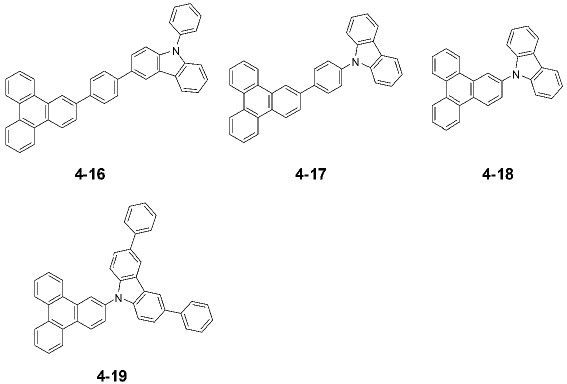

Therefore, the composition is applied to an emission layer of an organic optoelectric device, and thus good interface characteristics and hole and electron transport capability may be improved.

The first host compound and the second host compound may be included in a weight ratio of, for example about 1:10 to about 10:1.

Within the range, when an emission layer is formed in an appropriate weight ratio using electron transport capability of the first host compound and hole transport capability of the second host compound, bipolar characteristics may be realized and efficiency and life-span may be improved.

The composition may further include at least one host compound except the first host compound and the second host compound.

The composition may further include a dopant.

The dopant may be a red, green, or blue dopant, for example a phosphorescent dopant.

The dopant is mixed with the first host compound and the second host compound in a small amount to cause light emission, and may be generally a material such as a metal complex that emits light by multiple excitation into a triplet or more.

The dopant may be, for example an inorganic, organic, or organic/inorganic compound, and one or more kinds thereof may be used.

Examples of the phosphorescent dopant may be an organic metal compound including Ir, Pt, Os, Ti, Zr, Hf, Eu, Tb, Tm, Fe, Co, Ni, Ru, Rh, Pd, or a combination thereof.

The phosphorescent dopant may be, for example one of compounds represented by the following Chemical Formulae A to C, but is not limited thereto.

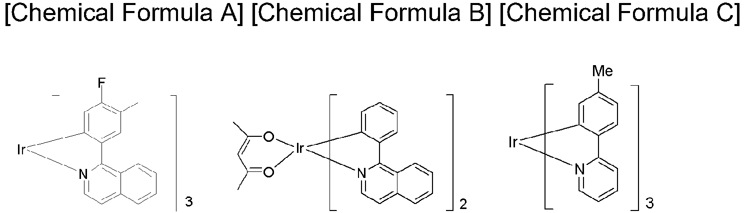

The composition may form a film using a dry film-forming method such as chemical vapor deposition.

Hereinafter, an organic optoelectric device to which the composition is applied is described.

The organic optoelectric device may be any device to convert electrical energy into photoenergy and vice versa without particular limitation, and may be, for example an organic photoelectric device, an organic light emitting diode, an organic solar cell, and an organic photo-conductor drum.

Herein, an organic light emitting diode as one example of an organic optoelectric device is described referring to drawings.

FIGS. 1 and 2 are cross-sectional views of each organic light emitting diode according to one embodiment.

Referring to FIG. 1, an organic optoelectric device 100 according to one embodiment includes an anode 120 and a cathode 110 facing each other and an organic layer 105 interposed between the anode 120 and cathode 110.

The anode 120 may be made of a conductor having a large work function to help hole injection, and may be for example metal, metal oxide and/or a conductive polymer.

The anode 120 may be, for example a metal nickel, platinum, vanadium, chromium, copper, zinc, gold, and the like or an alloy thereof; metal oxide such as zinc oxide, indium oxide, indium tin oxide (ITO), indium zinc oxide (IZO), and the like; a combination of metal and oxide such as ZnO and Al or SnO₂ and Sb; a conductive polymer such as poly(3-methylthiophene), poly(3,4-(ethylene-1,2-dioxy)thiophene) (PEDT), polypyrrole, and polyaniline, but is not limited thereto.

The cathode 110 may be made of a conductor having a small work function to help electron injection, and may be for example metal, metal oxide and/or a conductive polymer.

The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, LiO₂/Al, LiF/Ca, LiF/Al and BaF₂/Ca, but is not limited thereto.

The organic layer 105 may include an emission layer 130 including the above composition.

The emission layer 130 may include, for example the above composition.

Referring to FIG. 2, an organic light emitting diode 200 further includes a hole auxiliary layer 140 as well as an emission layer 130.

The hole auxiliary layer 140 may further increase hole injection and/or hole mobility between the anode 120 and emission layer 130 and block electrons.

The hole auxiliary layer 140 may be, for example a hole transport layer (HTL), a hole injection layer (HIL), and/or an electron blocking layer, and may include at least one layer.

In one embodiment of the present invention, an organic light emitting diode may further include an electron transport layer (ETL), an electron injection layer (EIL), a hole injection layer (HIL), and the like, as an organic thin layer 105 in FIG. 1 or FIG. 2.

The organic light emitting diodes 100 and 200 may be manufactured by forming an anode or a cathode on a substrate, forming an organic layer in accordance with a dry coating method such as evaporation, sputtering, plasma plating, and ion plating; and forming a cathode or an anode thereon.

The organic light emitting diode may be applied to an organic light emitting diode (OLED) display.

Hereinafter, the embodiments are illustrated in more detail with reference to examples.

These examples, however, are not in any sense to be interpreted as limiting the scope of the invention.

### Synthesis of Organic Compound

### Example 1: Synthesis of First Host Compound

A compound 1-705 as a specific example of the first compound was synthesized through two steps according to the following Reaction Scheme 1.

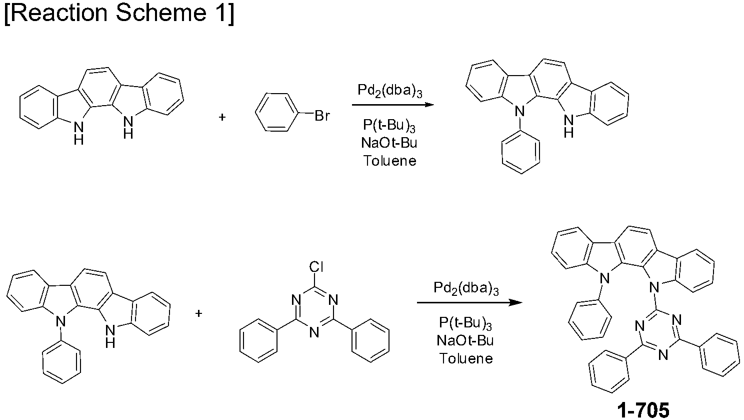

### First step; Synthesis of 11-phenyl-11,12-dihydroindolo[2,3-a]carbazole

78.35 g (305.69 mmol) of 11,12-dihydroindolo[2,3-a]carbazole, 26.8 mL (254.74 mmol) of bromobenzene, 26.93 g (280.22 mmol) of NaOt-Bu, and 7 g (7.64 mmmol) of Pd₂(dba)₃ were suspended in 1400 ml of toluene, 3.64 ml (15.28 mmol) of P(t-Bu)₃ was added thereto, and the mixture was refluxed and agitated for 12 hours.

Distilled water was added thereto for extraction, and an organic layer produced therein was filtered with silica gel.

After removing an organic solution therefrom, a solid product was recrystallized with dichloromethane and n-hexane, obtaining 46.2 g of 11-phenyl-11,12-dihydroindolo[2,3-a]carbazole (a yield : 55 %, LC Mass M+H⁺ = 333.13).

### Second step; Synthesis of Compound 1-705

46.2 g (138.99 mmol) of 11-phenyl-11,12-dihydroindolo[2,3-a]carbazole and 37.2 g (138.99 mmol) of 2-chloro-4,6-diphenyl-1,3,5-triazine, 16.03 g (166.79 mmol) of NaOt-Bu, and 7.63 g (8.34 mmmol) of Pd₂(dba)₃ were suspended in 500 ml of toluene, 12.2 ml (25.02 mmol) of P(t-Bu)₃ was added thereto, and the mixture was refluxed and agitated for 12 hours.

Subsequently, distilled water was added thereto for extraction, and an organic layer produced therein was filtered with silica gel.

After removing an organic solution therefrom, a solid product was dissolved in a small amount of dichloromethane, the solution was dropped in methanol for precipitation, and dichloromethane and n-hexane were used for recrystallization, obtaining 27.5 g of a compound 1-705 (a yield: 35 %, LC Mass M+H⁺ = 564.22).

### Examples 2 to 6: Preparation of Compound

A compound was synthesized according to the same method as Example 1 except for using two starting materials provided in the following Table instead of the starting material (corresponding to a starting material 1in the following Table 22) and A-1 (corresponding to a starting material 2 in the following Table 22) in Example 1.

**[Table 22]**

| Examples | Starting material 1 | Starting material 2 | Product | Yield (%) LC-Mass (M+H⁺) |
|---|---|---|---|---|
| 2 | 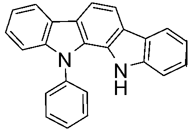 | 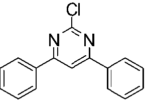 | 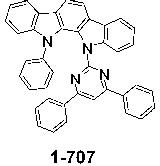 | 52 % |
| | | | | 563.22 |
| 3 | 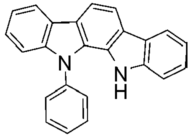 | 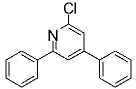 | 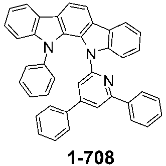 | 48 % |
| | | | | 562.23 |
| 4 | 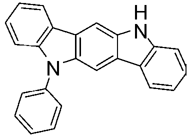 | 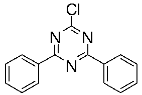 | 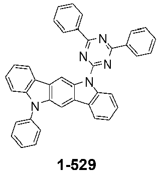 | 65 % |
| | | | | 564.21 |
| 5 | 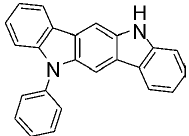 | 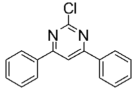 | 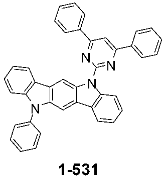 | 61 % |
| | | | | 563.22 |
| 6 | 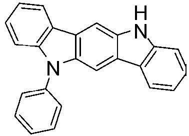 | 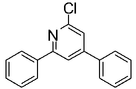 | 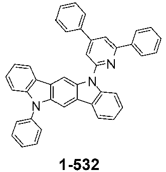 | 59 % |
| | | | | 562.22 |

### Examples 8 to 14: Preparation of Compound

The following compounds 2-944, 2-946, 2-949, 2-655, 2-658, 2-511, and 2-514 were synthesized according to the same method as Example 7 except for using starting materials provided in the following Table instead of the phenylcarbazolylbromide (corresponding to a starting material 1 in the following Table 23) and the phenylcarbazolylboronic acid (corresponding to a starting material 2 in the following Table 23) in Example 7.

**[Table 23]**

| Examples | Starting material 1 | Starting material 2 | Product | Yield (%) LC-Mass (M+H⁺) |
|---|---|---|---|---|
| 8 | 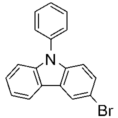 | 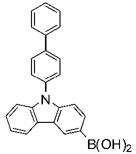 | 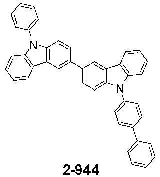 | 90 % |
| | | | | 561.23 |
| 9 | 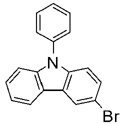 | 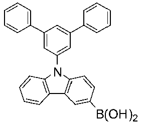 | 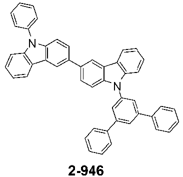 | 85 % |
| | | | | 637.27 |
| 10 | 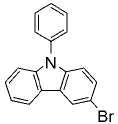 | 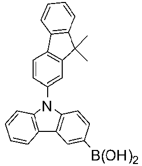 | 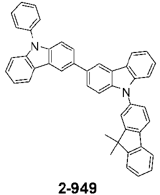 | 82 % |
| | | | | 601.26 |
| 11 | 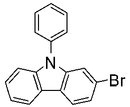 | 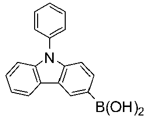 | 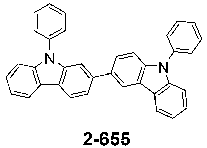 | 84 % |
| | | | | 485.19 |
| 12 | 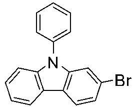 | 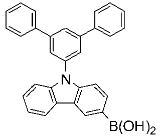 | 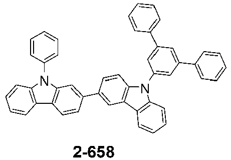 | 80 % |
| | | | | 637.28 |
| 13 | 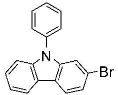 | 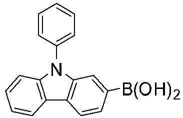 | 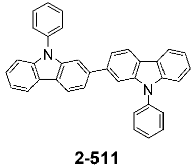 | 82 % |
| | | | | 485.19 |
| 14 | 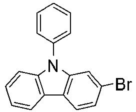 | 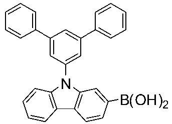 | 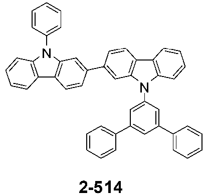 | 83 % |
| | | | | 637.27 |

### Example 15: Synthesis of Second Host Compound [Chemical Formula 5]

An intermediate product was synthesized through two steps as shown in the following Reaction Scheme 3 referring to KR 1247626 (Cheil Industries Inc., Registered Patent).

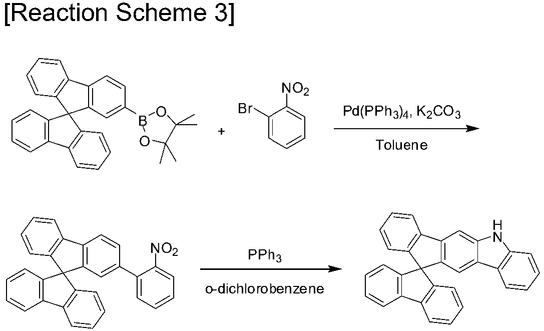

A compound 3-1 represented by Chemical Formula 5 as a specific example of a second host compound was synthesized according to the following Reaction Scheme 4.

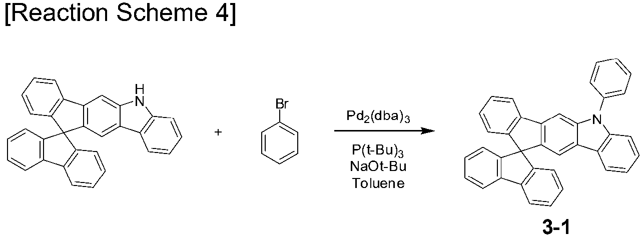

### First step; Synthesis of Compound 3-1

10 g (24.66 mmol) of spirofluorenoindenocarbazole, 2.59 mL (27.13 mmol) of bromobenzene, 4.74 g (49.32 mmol) of NaOt-Bu, and 0.23 g (0.25 mmmol) of Pd₂(dba)₃ were suspended in 100 ml of toluene, 0.6 ml (2.47 mmol) of P(t-Bu)₃ was added thereto, and the mixture was refluxed and agitated for 12 hours.

Dichloromethane and distilled water were used for extraction, and an organic layer produced therein was filtered with silica gel.

After removing an organic solution therein and performing silica gel column with hexane: dichloromethane = 8 : 2 (v/v), a solid product was recrystallized with dichloromethane and n-hexane, obtaining 10.9 g of a compound 3-1 (a yield : 92 %, LC Mass M+H⁺ = 482.20).

### Example 16 to 20: Preparation of Compound

The following compounds 3-2, 3-5, 3-7, 3-11, and 3-14 were obtained according to the same method as Example 15 except for using each starting material provided in the following Table instead of the spirofluorenoindenocarbazole (corresponding to a starting material 1 in the following Table24) and bromobenzene (corresponding to a starting material 2 in the following Table 24) in Example 15.

**[Table 24]**

| Examples | Starting material 1 | Starting material 2 | Product | Yield (%) LC-Mass (M+H⁺) |
|---|---|---|---|---|
| 16 | 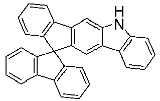 | 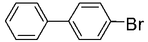 | 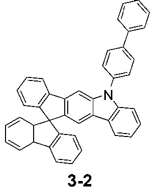 | 90 % |
| | | | | 558.21 |
| 17 | 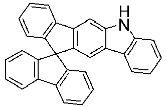 | 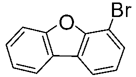 | 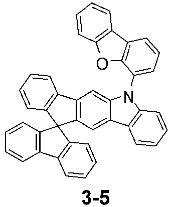 | 85 % |
| | | | | 572.20 |
| 18 | 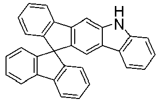 | 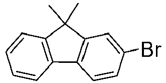 | 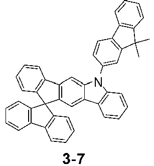 | 87 % |
| | | | | 598.25 |
| 19 | 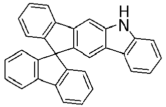 | 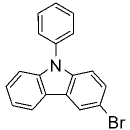 | 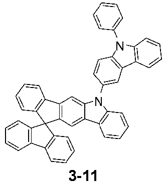 | 85 % |
| | | | | 647.25 |
| 20 | 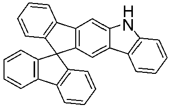 | 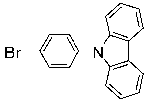 | 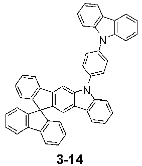 | 84 % |
| | | | | 647.24 |

### Example 21: Synthesis of Second Host Compound [Chemical Formulas 6 to 7]

A compound 4-1 represented by the following Reaction Scheme 5 as a specific example of a second host compound was synthesized as follows.

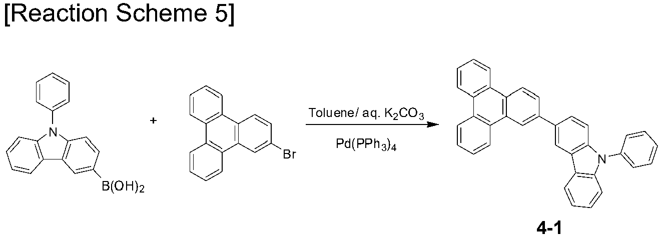

### First step; Synthesis of Compound 4-1

10 g (34.83 mmol) of phenylcarbazolylboronic acid, 11.77 g (38.31 mmol) of 2-bromotriphenylene, 14.44 g (104.49 mmol) of potassium carbonate, and 0.80 g (0.7 mmmol) of tetrakis-(triphenylphosphine)palladium (0) were suspended in 140 ml of toluene and 50 ml of distilled water, and the suspended solution was refluxed and agitated for 12 hours.

Dichloromethane and distilled water were used for extraction, and an organic layer produced therein was filtered with silica gel.

After removing an organic solution therefrom and performing silica gel column with hexane: dichloromethane = 7 : 3 (v/v), a solid product was recrystallized with dichloromethane and n-hexane, obtaining a compound 4-1 14.4 g (a yield : 88 %, LC Mass M+H⁺ = 470.19).

### Examples 22 to 25: Preparation of Compound

Compounds 4-2, 4-5, 4-8, and 4-15 were synthesized according to the same method as Example 21 except for using starting materials provided in the following Table instead of the phenylcarbazolylboronic acid (corresponding to a starting material 1 in the following Table 25) and the 2-bromotriphenylene (corresponding to a starting material 2 in the following Table 25) in Example 21.

**[Table 25]**

| Examples | Starting material 1 | Starting material 2 | Product | Yield (%) LC-Mass (M+H⁺) |
|---|---|---|---|---|
| 22 | 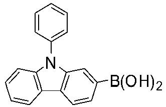 | 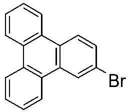 | 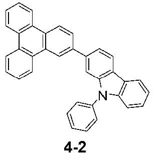 | 89 % |
| | | | | 470.18 |
| 23 | 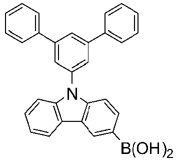 | 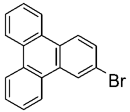 | 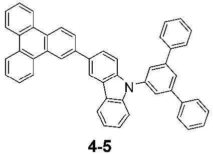 | 87 % |
| | | | | 622.25 |
| 24 | 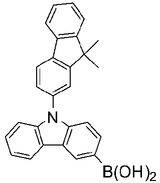 | 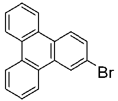 | 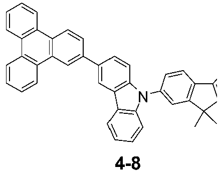 | 85 % |
| | | | | 586.25 |
| 25 | 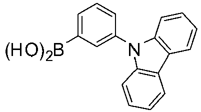 | 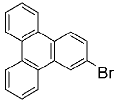 | 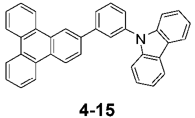 | 85 % |
| | | | | 470.19 |

### Example 26: Manufacture of Organic Light Emitting Diode

A 1000 Å-thick ITO was used as an anode, and a 1000 Å-thick aluminum (Al) was used as a cathode.

Specifically, an organic light emitting diode was manufactured by manufacturing the anode by cutting an ITO glass substrate having 15 Ω/cm² of sheet resistance into a size of 50 mm ×50 mm × 0.7 mm and cleaning with a ultrasonic wave in acetone, isopropyl alcohol, and pure water respectively for 5 minutes and with UV ozone for 30 minutes.

On the substrate upper, a 800 Å-thick hole transport layer (HTL) was formed by depositing N4,N4'-di(naphthalen-1-yl)-N4,N4'-diphenylbiphenyl-4,4'-diamine (NPB) (80 nm) with a vacuum degree of 650×10⁻⁷ Pa at a deposition speed ranging from 0.1 to 0.3 nm/s.

Subsequently, a 300 Å-thick emission layer was formed by vacuum-depositing the compound 1-705 obtained under the same vacuum deposition condition in Example 1 and the compound 2-943 according to Example 7 in a weight ratio of 1:1 and simultaneously, doping the host with Ir(ppy)3 as a phosphorescent dopant.

Herein, 10 wt% of the phosphorescent dopant was deposited by adjusting a speed of depositing the phosphorescent dopant based on 100 wt% of the total amount of the emission layer.

On the emission layer, a 50 Å-thick hole-blocking layer was formed by using bis(2-methyl-8-quinolinolate)-4-(phenylphenolato)aluminium (BAlq) under the same deposition condition.

Subsequently, a 250 Å-thick electron transport layer (ETL) was formed by depositing Alq3 under the same vacuum deposition condition.

LiF and Al were sequentially deposited to from a cathode on the electron transport layer (ETL), manufacturing an organic photoelectric device.

The organic photoelectric device has a structure of ITO/ NPB (80 nm)/ EML (a compound 1-705 (45 wt%) + a compound 2-943 (45 wt%) + Ir(PPy)3 (10 wt%), 30 nm)/ Balq (5 nm)/ Alq3 (25 nm)/ LiF (1 nm) / Al (100 nm).

### Example 27

An organic light emitting diode was manufactured according to the same method as Example 26 except for depositing a compound 1-705 and a compound 2-943 in a weight ratio of 4:1 instead of the compound 1-705 and the compound 2-943 in a weight ratio of 1:1 in Example 26.

### Example 28

An organic light emitting diode was manufactured according to the same method as Example 26 except for using a compound 1-705 and a compound 2-943 in a weight ratio of 1:4 instead of the compound 1-705 and the compound 2-943 in a weight ratio of 1:1 in Example 26.

### Example 29

An organic light emitting diode was manufactured according to the same method as Example 26 except for using a compound 1-705 and a compound 3-1 in a weight ratio of 1:1 instead of the compound 1-705 and the compound 2-943 in a weight ratio of 1:1 in Example 26.

### Example 30

An organic light emitting diode was manufactured according to the same method as Example 26 except for using a compound 1-705 and a compound 4-1 in a weight ratio of 1:1 instead of the compound 1-705 and the compound 2-943 in a weight ratio of 1:1 in Example 26.

### Example 31

An organic light emitting diode was manufactured according to the same method as Example 30 except for using a compound 1-705 and a compound 4-1 in a weight ratio of 4:1 instead of the compound 1-705 and the compound 4-1 in a weight ratio of 1:1 in Example 30.

### Example 32

An organic light emitting diode was manufactured according to the same method as Example 30 except for using a compound 1-705 and a compound 4-1 in a weight ratio of 1:4 instead of the compound 1-705 and the compound 4-1 in a weight ratio of 1:1 in Example 30.

### Example 33

An organic light emitting diode was manufactured according to the same method as Example 30 except for using a compound 1-705 and a compound 4-15 in a weight ratio of 1:1 instead of the compound 1-705 and the compound 4-1 in a weight ratio of 1:1 in Example 30.

### Comparative Example 1

An organic light emitting diode was manufactured according to the same method as Example 26 except for only depositing a compound 1-705 as a host instead of the compound 1-705 and the compound 2-943 in a weight ratio of 1:1 in Example 26.

### Comparative Example 2

An organic light emitting diode was manufactured according to the same method as Example 26 except for only depositing a compound 2-943 as a host instead of the compound 1-705 and the compound 2-943 in a weight ratio of 1:1 in Example 26.

### Comparative Example 3

An organic light emitting diode was manufactured according to the same method as Example 29 except for only depositing a compound 3-1 as a host instead of the compound 1-705 and the compound 3-1 in a weight ratio of 1:1 in Example 29.

### Comparative Example 4

An organic light emitting diode was manufactured according to the same method as Example 30 except for only depositing a compound 4-1 as a host instead of the compound 1-705 and the compound 4-1 in a weight ratio of 1:1 in Example 30.

### Comparative Example 5

An organic light emitting diode was manufactured according to the same method as Example 26 except for depositing a compound 1-705 and a comparative compound having the following structure formula, HOST-1, in a weight ratio of 1:1 instead of the compound 1-705 and the compound 2-943 in a weight ratio of 1:1 in Example 26.

### HOST-1

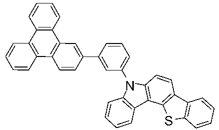

### Evaluation

Current density and luminance changes depending on a voltage and luminous efficiency of each organic light emitting diode according to Examples 1 to 8 and Comparative Examples 1 to 5 were measured.

The measurements were specifically performed in the following method, and the results were provided in the following Table 26.

### (1) Measurement of Current density Change depending on Voltage Change

Current values flowing in the unit device of the manufactured organic light emitting diodes were measured for, while increasing the voltage from 0V to 10V using a current-voltage meter (Keithley 2400), and the measured current values were divided by an area to provide the results.

### (2) Measurement of Luminance Change depending on Voltage Change

Luminance of the manufactured organic light emitting diodes was measured for luminance, while increasing the voltage from 0V to 10V using a luminance meter (Minolta Cs-1000A).

### (3) Measurement of Luminous Efficiency

Current efficiency (cd/A) at the same current density (10 mA/cm²) were calculated by using the luminance, current density, and voltages (V) from the items 1) current density change depending on voltage change and 2) luminance change depending on voltage change.

### (4) Measurement of Life-span

Luminance (cd/m²) was maintained at 5000 cd/m² and a time at current efficiency (cd/A) decreases to 95% was measured.

**[Table 26]**

| | Compound used in emission layer | Driving voltage (V) | Color (EL color) | Efficiency (cd/A) | 95% life-span (h) @ 5000 cd/m² |
|---|---|---|---|---|---|
| Example 26 | 1-705 + 2-943 1:1 | 3.8 | Green | 52.9 | 260 |
| Example 27 | 1-705 + 2-943 4:1 | 3.9 | Green | 37.1 | 330 |
| Example 28 | 1-705 + 2-943 1:4 | 5.3 | Green | 24.5 | 40 |
| Example 29 | 1-705 + 3-1 1:1 | 5.5 | Green | 25.2 | 70 |
| Example 30 | 1-705 + 4-1 1:1 | 4.3 | Green | 26.3 | 340 |
| Example 31 | 1-705 + 4-1 4:1 | 4.3 | Green | 36.8 | 360 |
| Example 32 | 1-705 + 4-1 1:4 | 5.1 | Green | 45.3 | 300 |
| Example 33 (comparative) | 1-705 + 4-15 1:1 | 4.8 | Green | 24.1 | 230 |
| Comparative Example 1 | 1-705 | 4.3 | Green | 24.2 | 240 |
| Comparative Example 2 | 2-943 | 7.1 | Green | 1.7 | - |
| Comparative Example 3 | 3-1 | 8.5 | Green | 25.6 | - |
| Comparative Example 4 | 4-1 | 7.9 | Green | 16.5 | 22 |
| Comparative Example 5 | 1-705 + HOST-1 1:1 | 5.0 | Green | 22.9 | 200 |

From the Table 26, the organic light emitting diodes according to Examples 26 to 33 showed remarkably improved luminous efficiency and life-span compared with the organic light emitting diodes according to Comparative Examples 1 to 3.

Specifically, the organic light emitting diodes according to Example 30 to Example 32 showed improved efficiency and superbly improved life-span compared with each comparative example, Comparative Examples 1 and 4.

These efficiency and life-span may be improved due to bipolar characteristics of the hole transportable host and the electron transportable host.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

Therefore, the aforementioned embodiments should be understood to be exemplary but not limiting the present invention in any way.

## Claims

1. A composition, comprising
a first host compound including moieties represented by the Chemical Formulae 1 to 3 that are sequentially bonded with each other, 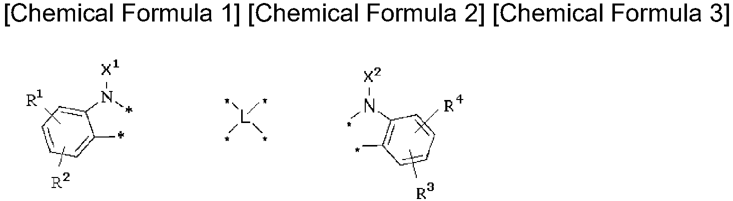
wherein, in the Chemical Formulae 1 to 3,
X¹ is *-Y¹-ET,
X² is *-Y²-Ar¹,
ET is a substituent capable of accepting an electron when electric field is applied,
Ar¹ is a substituted or unsubstituted C6 to C30 aryl group, a substituted or unsubstituted C2 to C30 heteroaryl group, or a combination thereof,
Y¹ and Y² are each independently a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof,
L is a substituted or unsubstituted C2 or C3 alkenylene group or a substituted or unsubstituted C6 to C20 arylene group, and
R¹ to R⁴ are each independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.
and
a second host compound represented by one of the Chemical Formula 5 to 6: 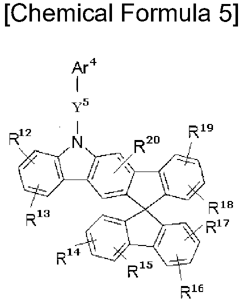
wherein, in the Chemical Formula 5,
Ar⁴ is a substituted or unsubstituted C6 to C30 aryl group, a substituted or unsubstituted C2 to C30 heteroaryl group, or a combination thereof,
Y⁵ is a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof, and
R¹² to R²⁰ are independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof. 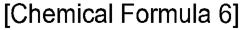 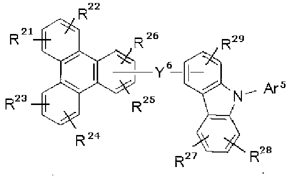
wherein, in the Chemical Formula 6
Ar⁵ is a substituted or unsubstituted C6 to C30 aryl group, a substituted or unsubstituted C2 to C30 heteroaryl group, or a combination thereof,
Y⁶ is independently a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof, and
R²¹ to R²⁹ are independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

2. The composition of claim 1, wherein the ET is represented by the Chemical Formula 1a: 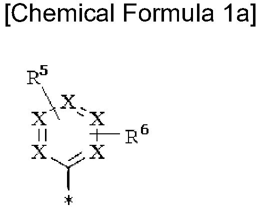 wherein, in the Chemical Formula 1a,
X is N, C or CR^{a},
at least one of X is N, and
R⁵, R⁶ and R^{a} are each independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

3. The composition of claim 1, wherein the ET is one of the substituents listed in the Group 1: 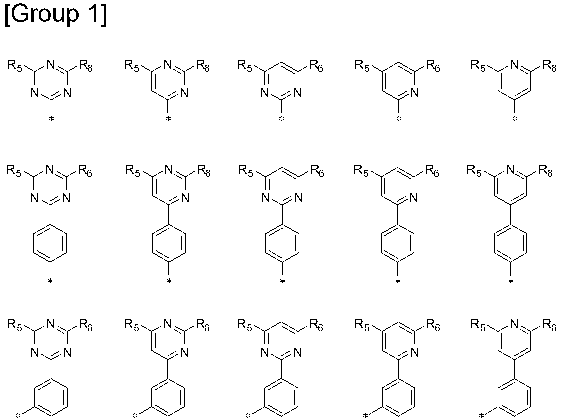 wherein, in the Group 1,
R⁵ and R⁶ are each independently hydrogen, deuterium, a substituted or unsubstituted C1 to C20 alkyl group, a substituted or unsubstituted C6 to C30 aryl group, a substituted or unsubstituted C2 to C30 heteroaryl group, or a combination thereof.

4. The composition of any of the claims 1 to 3, wherein the X¹ is one of the substituents listed in the Group 2:

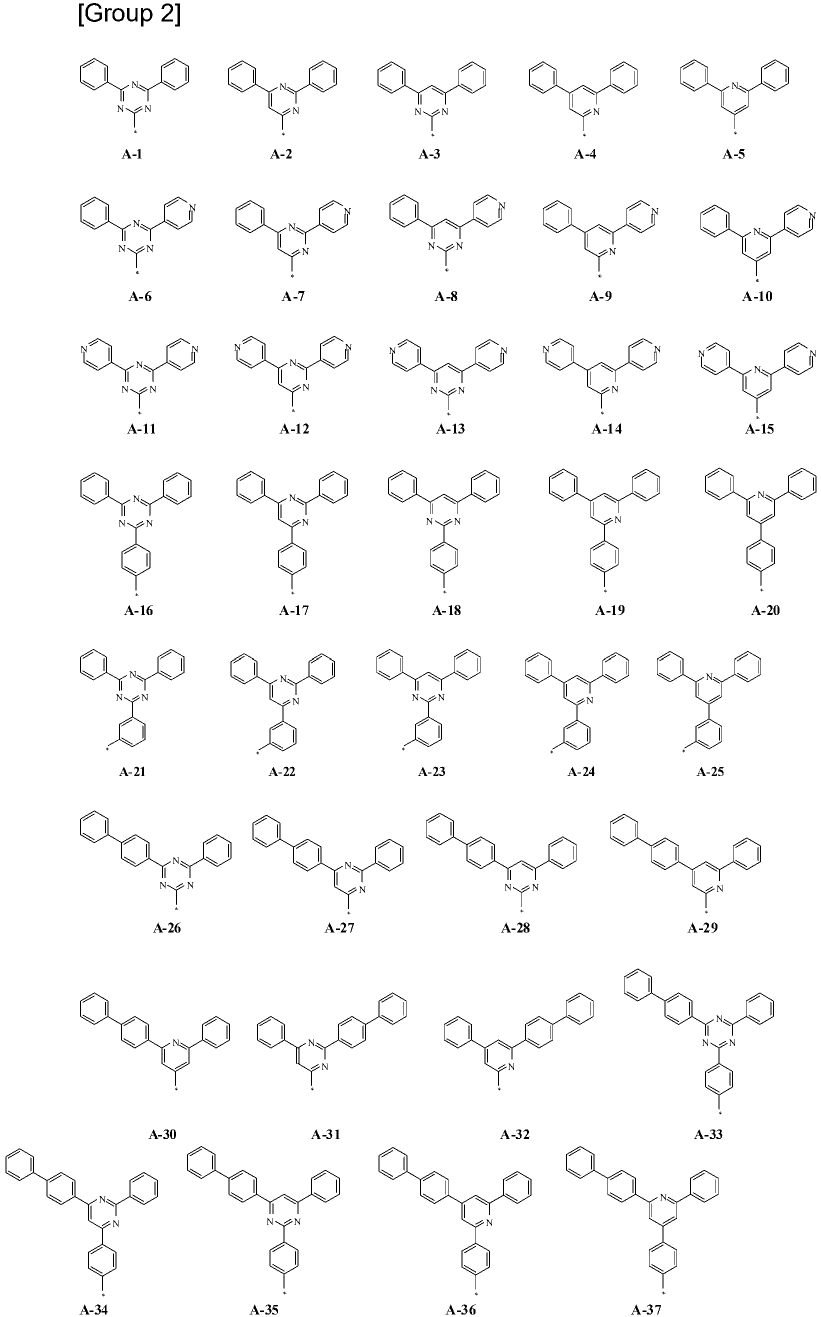

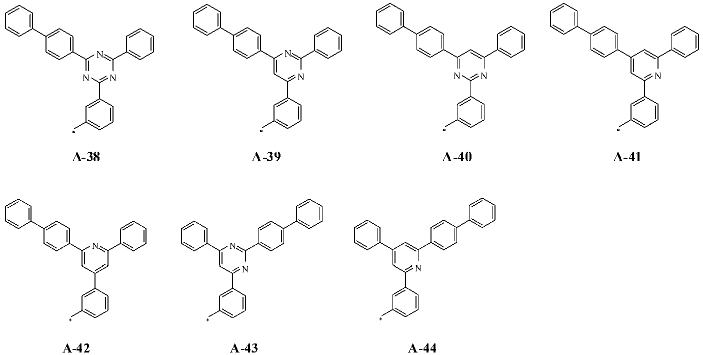

5. The composition of any of the claims 1 to 4, wherein the moiety represented by the Chemical Formula 2 is represented by one of the following Chemical Formulae 2-1 to 2-3:

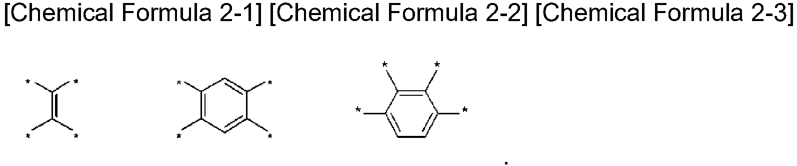

6. The composition of any of the claims 1 to 5, wherein the Ar¹ is a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted carbazolyl group, a substituted or unsubstituted benzofuranyl group, a substituted or unsubstituted benzothiophenyl group, a substituted or unsubstituted fluorenyl group, a substituted or unsubstituted dibenzothiophenyl group, a substituted or unsubstituted dibenzofuranyl group, or a combination thereof.

7. The composition of any of the claims 1 to 6, wherein the X² is one of the substituents listed in the Group 3:

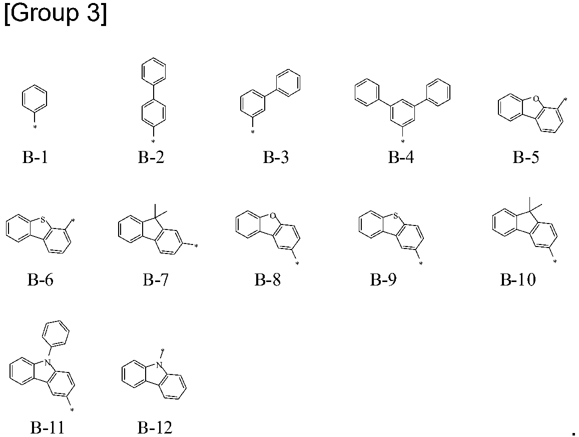

8. The composition of claim 1, wherein the first host compound is one of compounds listed in the Group 4: 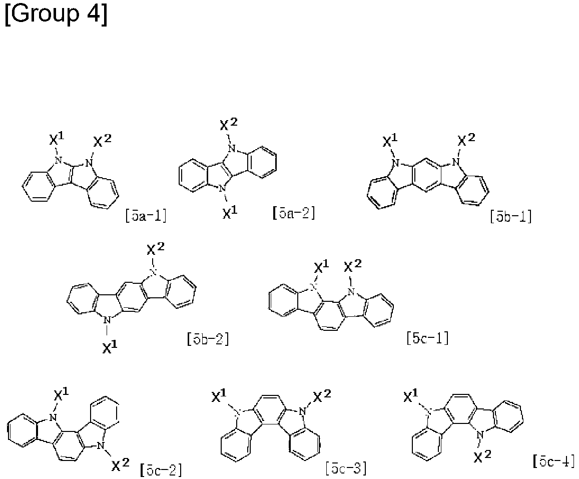 wherein, in the Group 4,
X¹ is *-Y¹-ET,
X² is *-Y²-Ar¹,
ET is a substituent capable of accepting an electron when electric field is applied,
Ar¹ is a substituted or unsubstituted C6 to C30 aryl group, a substituted or unsubstituted C2 to C30 heteroaryl group, or a combination thereof, and
Y¹ and Y² are each independently a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof.

9. The composition of any of the claims 1 to 8, wherein Ar⁴ is a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted carbazolyl group, a substituted or unsubstituted benzofuranyl group, a substituted or unsubstituted benzothiophenyl group, a substituted or unsubstituted fluorenyl group, a substituted or unsubstituted dibenzofuranyl group, a substituted or unsubstituted dibenzothiophenyl group, or a combination thereof.

10. The composition of any of the claims 1 to 9, wherein the second host compound is one of compounds listed in the Group 6:

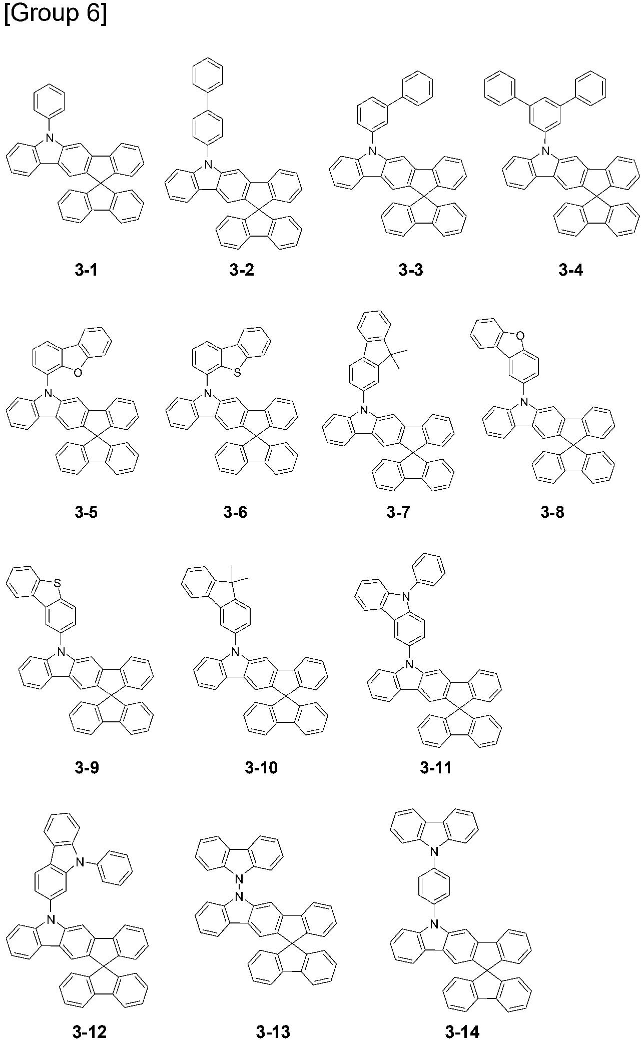

11. The composition of any of the claims 1 to 10, wherein Ar⁵ is a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted carbazolyl group, a substituted or unsubstituted benzofuranyl group, a substituted or unsubstituted benzothiophenyl group, a substituted or unsubstituted fluorenyl group, a substituted or unsubstituted dibenzothiophenyl group, a substituted or unsubstituted dibenzofuranyl group, or a combination thereof.

12. The composition of any of the claims 1 to 11, wherein the second host compound is one of compounds listed in the following Group 7:

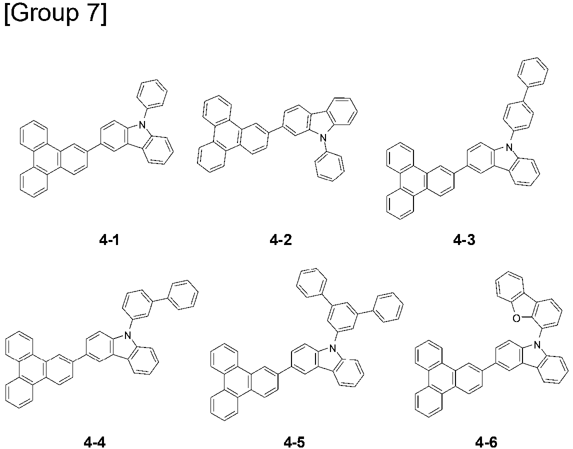

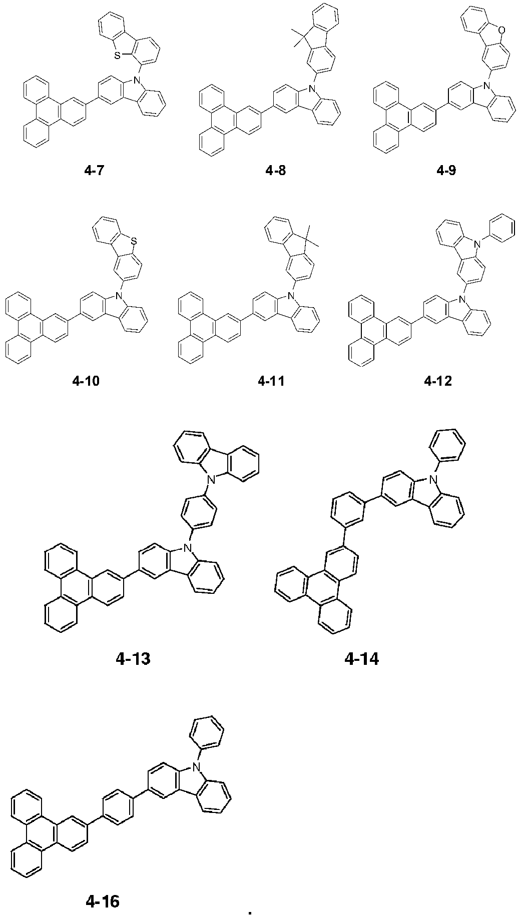

13. The composition of any of the claims 1 to 12, wherein first host compound and the second host compound are included in a weight ratio of about 1:10 to about 10:1.

14. An organic optoelectric device, comprising
an anode and a cathode facing each other,
at least one organic layer interposed between the anode and the cathode,
wherein the organic layer comprises the composition of any of the claims 1 to 13.

15. A display device comprising the organic optoelectric device of claim 14.

## Patentansprüche

1. Zusammensetzung, aufweisend:
eine erste Wirtsverbindung, die durch die chemischen Formeln 1 bis 3 dargestellte Struktureinheiten enthält, die sequentiell miteinander verbunden sind, 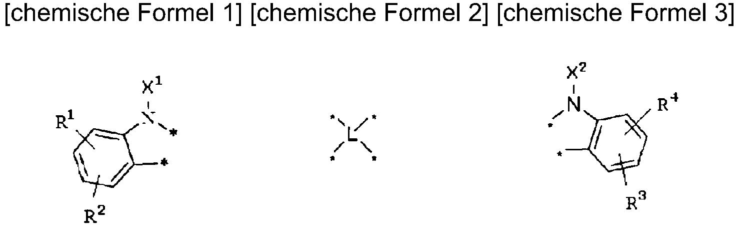
wobei in den chemischen Formeln 1 bis 3
X¹ *-Y¹-ET ist,
X² *-Y²-Ar¹ ist,
ET ein Substituent ist, der dazu geeignet ist, bei Anlegen eines elektrischen Feldes ein Elektron aufzunehmen,
Ar¹ eine substituierte oder unsubstituierte C6- bis C30-Arylgruppe, eine substituierte oder unsubstituierte C2- bis C30-Heteroarylgruppe oder eine Kombination davon ist,
Y¹ und Y² jeweils unabhängig voneinander eine Einfachbindung, eine substituierte oder unsubstituierte C1- bis C20-Alkylengruppe, eine substituierte oder unsubstituierte C2- bis C20-Alkenylengruppe, eine substituierte oder unsubstituierte C6- bis C30-Arylengruppe, eine substituierte oder unsubstituierte C2- bis C30-Heteroarylengruppe oder eine Kombination davon sind,
L eine substituierte oder unsubstituierte C2- oder C3-Alkenylengruppe oder eine substituierte oder unsubstituierte C6- bis C20-Arylengruppe ist, und
R¹ bis R⁴ jeweils unabhängig voneinander Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C2- bis C30-Heteroarylgruppe oder eine Kombination davon sind;
und
eine zweite Wirtsverbindung, die durch eine der chemischen Formeln 5 bis 6 dargestellt ist: 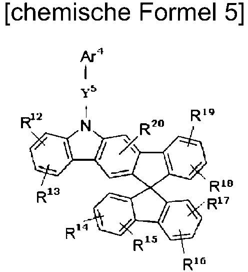
wobei in der chemischen Formel 5
Ar⁴ eine substituierte oder unsubstituierte C6- bis C30-Arylgruppe, eine substituierte oder unsubstituierte C2- bis C30-Heteroarylgruppe oder eine Kombination davon ist,
Y⁵ eine Einfachbindung, eine substituierte oder unsubstituierte C1- bis C20-Alkylengruppe, eine substituierte oder unsubstituierte C2- bis C20-Alkenylengruppe, eine substituierte oder unsubstituierte C6- bis C30-Arylengruppe, eine substituierte oder unsubstituierte C2- bis C30-Heteroarylengruppe oder eine Kombination davon ist, und
R¹² bis R²⁰ unabhängig voneinander Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C2- bis C30-Heteroarylgruppe oder eine Kombination davon sind; 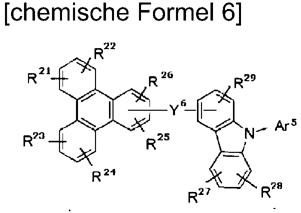
wobei in der chemischen Formel 6 Ar⁵ eine substituierte oder unsubstituierte C6- bis C30-Arylgruppe, eine substituierte oder unsubstituierte C2- bis C30-Heteroarylgruppe oder eine Kombination davon ist,
Y⁶ eine Einfachbindung, eine substituierte oder unsubstituierte C1- bis C20-Alkylengruppe, eine substituierte oder unsubstituierte C2- bis C20-Alkenylengruppe, eine substituierte oder unsubstituierte C6- bis C30-Arylengruppe, eine substituierte oder unsubstituierte C2- bis C30-Heteroarylengruppe oder eine Kombination davon ist, und
R²¹ bis R²⁹ unabhängig voneinander Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C2- bis C30-Heteroarylgruppe oder eine Kombination davon sind.

2. Zusammensetzung nach Anspruch 1, wobei ET durch die chemische Formel 1a dargestellt ist: 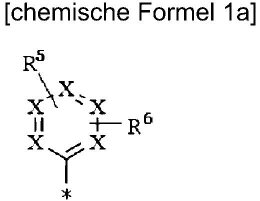 wobei in der chemischen Formel 1a
X N, C oder CR^{a} ist,
mindestens eine der Komponenten X N ist, und
R⁵, R⁶ und R^{a} jeweils unabhängig voneinander Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C2- bis C30-Heteroarylgruppe oder eine Kombination davon sind.

3. Zusammensetzung nach Anspruch 1, wobei ET einer der in Gruppe 1 aufgeführten Substituenten ist: 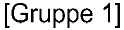 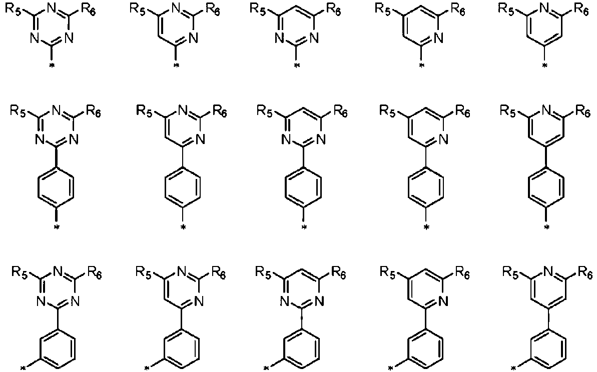 wobei in Gruppe 1
R⁵ und R⁶ jeweils unabhängig voneinander Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C2- bis C30-Heteroarylgruppe oder eine Kombination davon sind.

4. Zusammensetzung nach einem der Ansprüche 1 bis 3, wobei X¹ einer der in Gruppe 2 aufgeführten Substituenten ist:

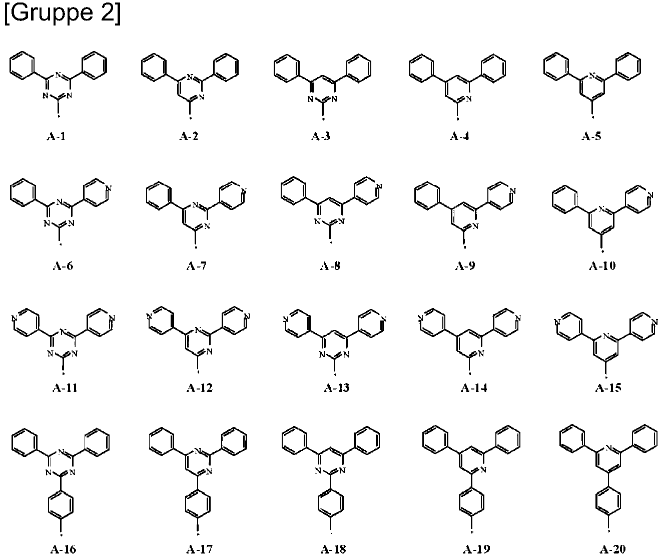

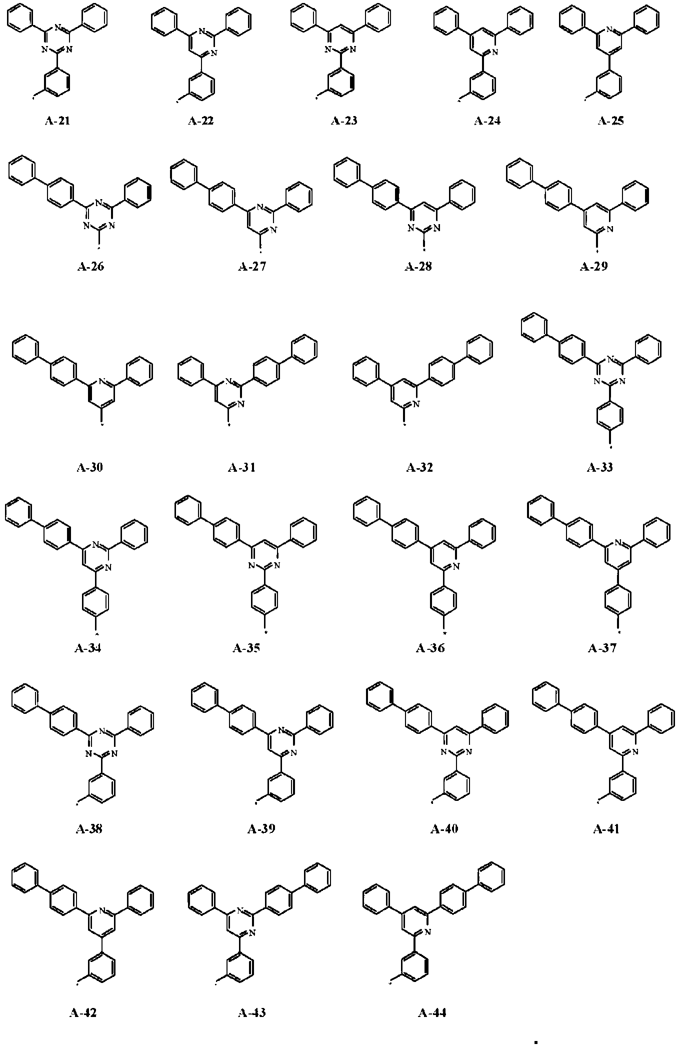

5. Zusammensetzung nach einem der Ansprüche 1 bis 4, wobei die durch die chemische Formel 2 dargestellte Struktureinheit durch eine der folgenden chemischen Formeln 2-1 bis 2-3 dargestellt wird:

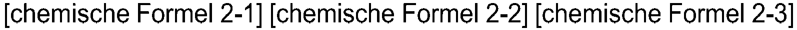

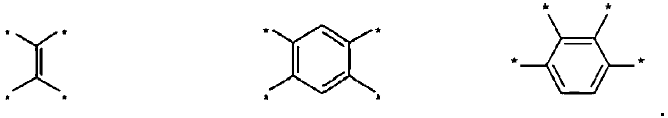

6. Zusammensetzung nach einem der Ansprüche 1 bis 5, wobei Ar¹ eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Carbazolylgruppe, eine substituierte oder unsubstituierte Benzofuranylgruppe, eine substituierte oder unsubstituierte Benzothiophenylgruppe, eine substituierte oder unsubstituierte Fluorenylgruppe, eine substituierte oder unsubstituierte Dibenzothiophenylgruppe, eine substituierte oder unsubstituierte Dibenzofuranylgruppe oder eine Kombination davon ist.

7. Zusammensetzung nach einem der Ansprüche 1 bis 6, wobei X² einer der in Gruppe 3 aufgeführten Substituenten ist:

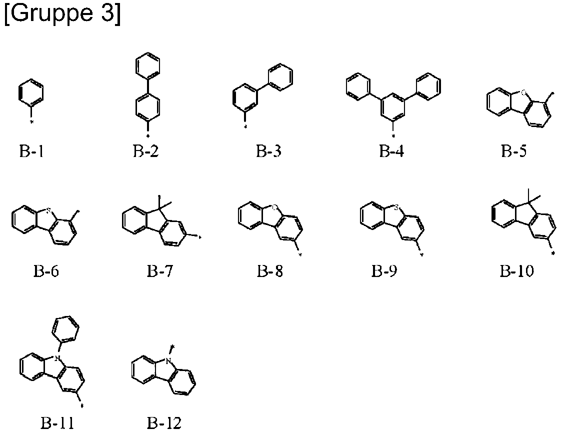

8. Zusammensetzung nach Anspruch 1, wobei die erste Wirtsverbindung eine der in Gruppe 4 aufgeführten Verbindungen ist: 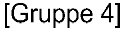 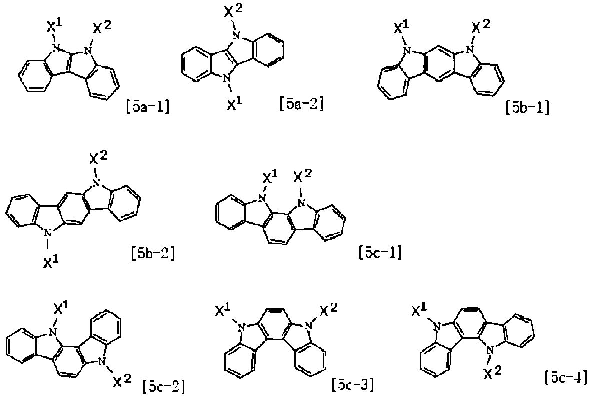 wobei in Gruppe 4
X¹ *-Y^{I}-ET ist,
X² *-Y²-Ar¹ ist,
ET ein Substituent ist, der in der Lage ist, bei Anlegen eines elektrischen Feldes ein Elektron aufzunehmen,
Ar¹ eine substituierte oder unsubstituierte C6- bis C30-Arylgruppe, eine substituierte oder unsubstituierte C2- bis C30-Heteroarylgruppe oder eine Kombination davon ist, und
Y¹ und Y² jeweils unabhängig voneinander eine Einfachbindung, eine substituierte oder unsubstituierte C1- bis C20-Alkylengruppe, eine substituierte oder unsubstituierte C2- bis C20-Alkenylengruppe, eine substituierte oder unsubstituierte C6- bis C30-Arylengruppe, eine substituierte oder unsubstituierte C2- bis C30-Heteroarylengruppe oder eine Kombination davon sind.

9. Zusammensetzung nach einem der Ansprüche 1 bis 8, wobei Ar⁴ eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Carbazolylgruppe, eine substituierte oder unsubstituierte Benzofuranylgruppe, eine substituierte oder unsubstituierte Benzothiophenylgruppe, eine substituierte oder unsubstituierte Fluorenylgruppe, eine substituierte oder unsubstituierte Dibenzofuranylgruppe, eine substituierte oder unsubstituierte Dibenzothiophenylgruppe oder eine Kombination davon ist.

10. Zusammensetzung nach einem der Ansprüche 1 bis 9, wobei die zweite Wirtsverbindung eine der in Gruppe 6 aufgeführten Verbindungen ist:

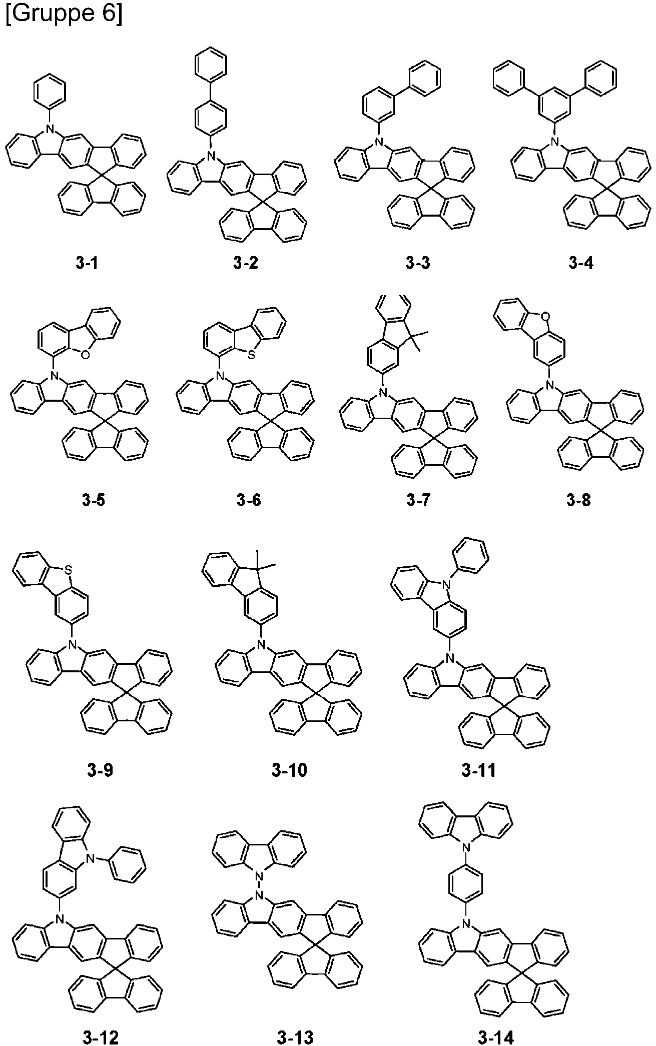

11. Zusammensetzung nach einem der Ansprüche 1 bis 10, wobei Ar⁵ eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Carbazolylgruppe, eine substituierte oder unsubstituierte Benzofuranylgruppe, eine substituierte oder unsubstituierte Benzothiophenylgruppe, eine substituierte oder unsubstituierte Fluorenylgruppe, eine substituierte oder unsubstituierte Dibenzothiophenylgruppe, eine substituierte oder unsubstituierte Dibenzofuranylgruppe oder eine Kombination davon ist.

12. Zusammensetzung nach einem der Ansprüche 1 bis 11, wobei die zweite Wirtsverbindung eine der in der folgenden Gruppe 7 aufgeführten Verbindungen ist:

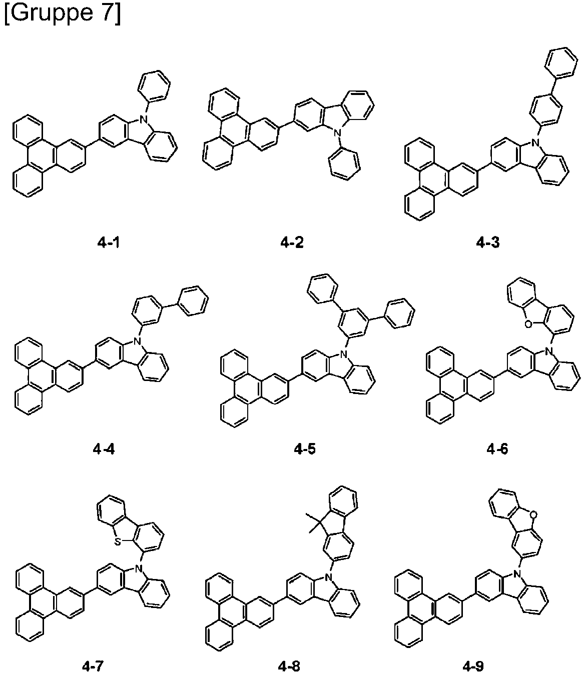

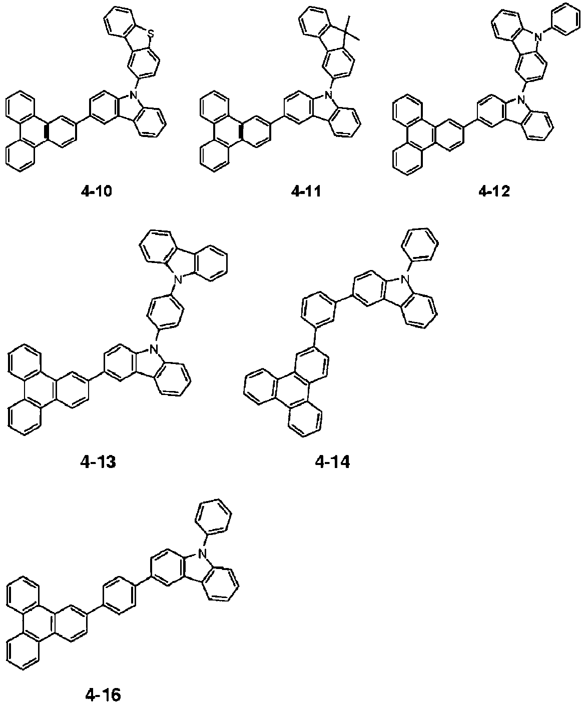

13. Zusammensetzung nach einem der Ansprüche 1 bis 12, wobei die erste Wirtsverbindung und die zweite Wirtsverbindung in einem Gewichtsverhältnis von etwa 1:10 bis etwa 10:1 enthalten sind.

14. Organische optoelektrische Vorrichtung, aufweisend:
eine Anode und eine Kathode, die einander zugewandt sind;
mindestens eine zwischen der Anode und der Kathode angeordnete organische Schicht,
wobei die organische Schicht die Zusammensetzung nach einem der Ansprüche 1 bis 13 enthält.

15. Anzeigevorrichtung, aufweisend die organische optoelektrische Vorrichtung nach Anspruch 14.

## Revendications

1. Composition, comprenant
un premier composé hôte comprenant des fragments représentés par les Formules Chimiques 1 à 3 qui sont reliés séquentiellement l'un à l'autre, 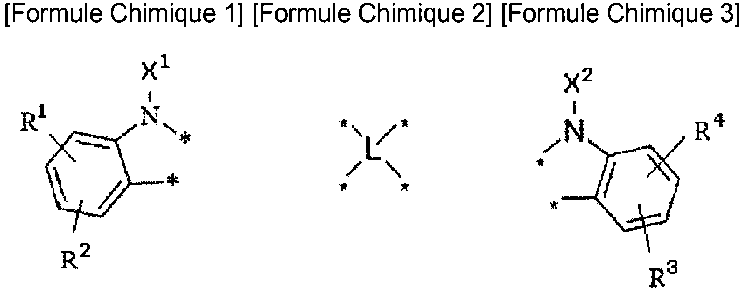
dans laquelle, dans les Formules Chimiques 1 à 3,
X¹ est *-Y¹-ET,
X² est *-Y²-Ar¹ ,
ET représente un substituant capable d'accepter un électron lorsqu'un champ électrique est appliqué,
Ar¹ représente un groupe aryle en C6 à C30 substitué ou non substitué, un groupe hétéroaryle en C2 à C30 substitué ou non substitué, ou une combinaison de ceux-ci,
Y¹ et Y² représentent chacun indépendamment une liaison simple, un groupe alkylène en C1 à C20 substitué ou non substitué, un groupe alcénylène en C2 à C20 substitué ou non substitué, un groupe arylène en C6 à C30 substitué ou non substitué, un groupe hétéroarylène en C2 à C30 substitué ou non, ou une combinaison de ceux-ci,
L représente un groupe alcénylène en C2 ou C3 substitué ou non substitué ou un groupe arylène en C6 à C20 substitué ou non substitué, et
R¹ à R⁴ représentent chacun indépendamment un hydrogène, un deutérium, un groupe alkyle en C1 à C20 substitué ou non substitué, un groupe aryle en C6 à C30 substitué ou non substitué, un groupe hétéroaryle en C2 à C30 substitué ou non substitué, ou une combinaison de ceux-ci.
et
un deuxième composé hôte représenté par l'une des Formules Chimiques 5 à 6 : 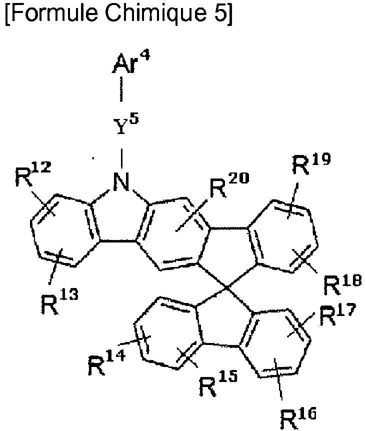
dans laquelle, dans la Formule Chimique 5,
Ar⁴ représente un groupe aryle en C6 à C30 substitué ou non substitué, un groupe hétéroaryle en C2 à C30 substitué ou non, ou une combinaison de ceux-ci,
Y⁵ représente une liaison simple, un groupe alkylène en C1 à C20 substitué ou non substitué, un groupe alcénylène en C2 à C20 substitué ou non substitué, un groupe arylène en C6 à C30 substitué ou non substitué, un groupe hétéroarylène en C2 à C30 substitué ou non substitué, ou une combinaison de ceux-ci, et
R¹² à R ²⁰ représentent indépendamment un hydrogène, un deutérium, un groupe alkyle en C1 à C20 substitué ou non substitué, un groupe aryle en C6 à C30 substitué ou non substitué, un groupe hétéroaryle en C2 à C30 substitué ou non substitué, ou une combinaison de ceux-ci. 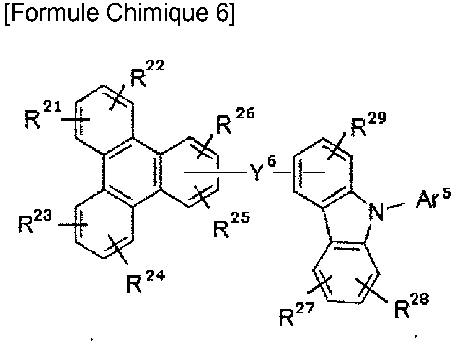
dans laquelle, dans la Formule Chimique 6
Ar⁵ représente un groupe aryle en C6 à C30 substitué ou non substitué, un groupe hétéroaryle en C2 à C30 substitué ou non substitué, ou une combinaison de ceux-ci,
Y⁶ représente indépendamment une liaison simple, un groupe alkylène en C1 à C20 substitué ou non substitué, un groupe alcénylène en C2 à C20 substitué ou non substitué, un groupe arylène en C6 à C30 substitué ou non substitué, un groupe hétéroarylène C2 à C30 substitué ou non substitué, ou une combinaison de ceux-ci, et
R²¹ à R²⁹ représentent indépendamment un hydrogène, un deutérium, un groupe alkyle en C1 à C20 substitué ou non substitué, un groupe aryle en C6 à C30 substitué ou non substitué, un groupe hétéroaryle en C2 à C30 substitué ou non substitué, ou une combinaison de ceux-ci.

2. Composition selon la revendication 1, dans laquelle ET est représenté par la Formule Chimique 1a : 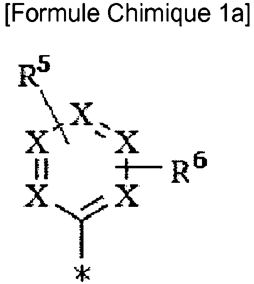 dans laquelle, dans la Formule Chimique 1a,
X représente N, C ou CR^{a},
au moins un de X est N, et
R⁵ , R ⁶ et R^{a} représentent chacun indépendamment un hydrogène, un deutérium, un groupe alkyle en C1 à C20 substitué ou non substitué, un groupe aryle en C6 à C30 substitué ou non substitué, un groupe hétéroaryle en C2 à C30 substitué ou non substitué, ou une combinaison de ceux-ci.

3. Composition selon la revendication 1, dans laquelle ET est l'un des substituants listés dans le Groupe 1 : 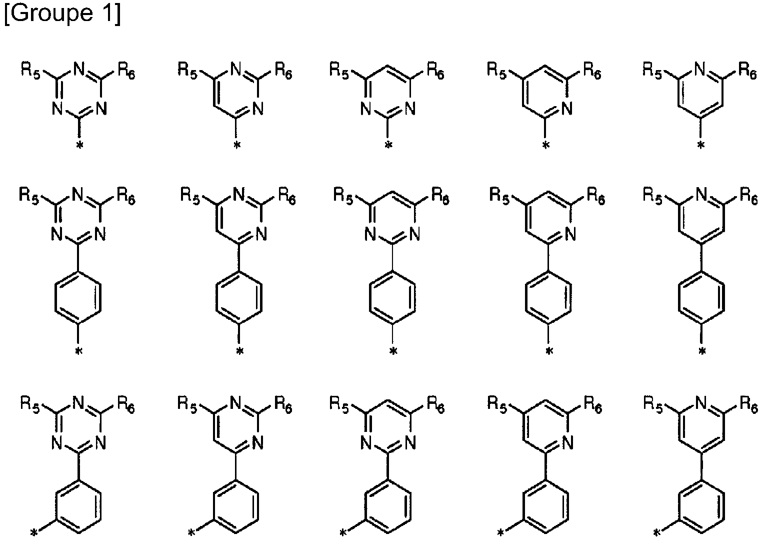 dans laquelle, dans le Groupe 1,
R⁵ et R⁶ représentent chacun indépendamment un hydrogène, un deutérium, un groupe alkyle en C1 à C20 substitué ou non substitué, un groupe aryle en C6 à C30 substitué ou non substitué, un groupe hétéroaryle en C2 à C30 substitué ou non substitué, ou une combinaison de ceux-ci.

4. Composition selon l'une quelconque des revendications 1 à 3, dans laquelle X¹ est l'un des substituants listés dans le Groupe 2 :

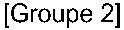

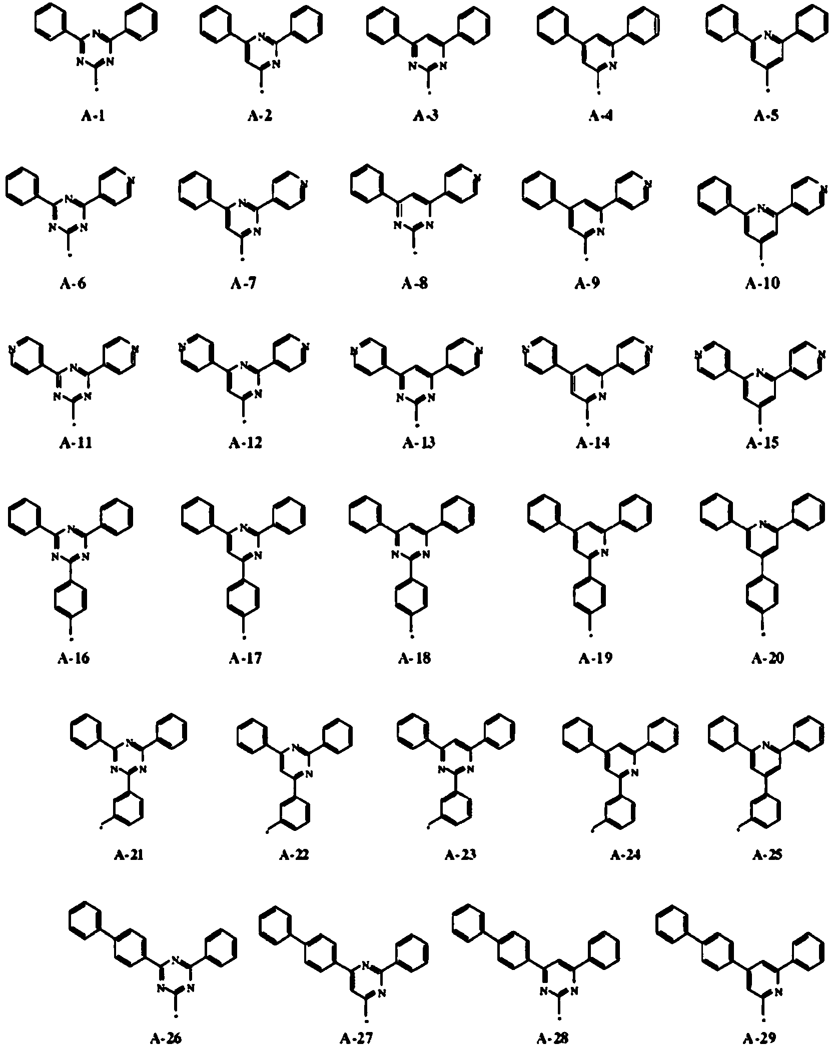

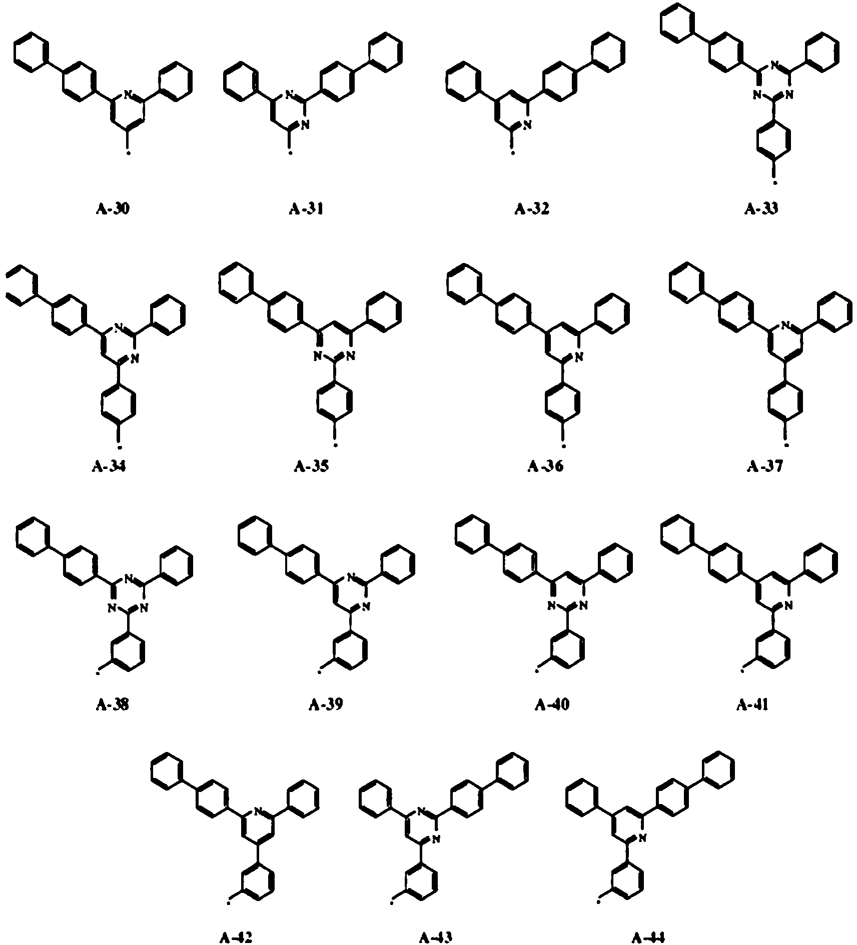

5. Composition selon l'une quelconque des revendications 1 à 4, dans laquelle le fragment représenté par la Formule Chimique 2 est représenté par l'une des Formules Chimiques suivantes 2-1 à 2-3 :

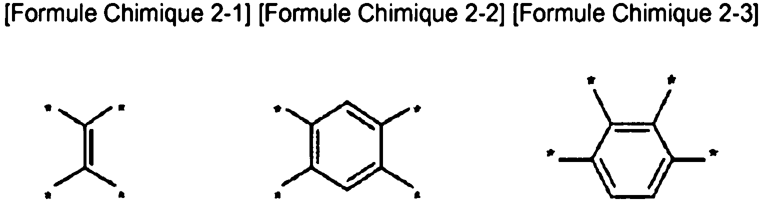

6. Composition selon l'une quelconque des revendications 1 à 5, dans laquelle Ar¹ est un groupe phényle substitué ou non substitué, un groupe biphényle substitué ou non substitué, un groupe terphényle substitué ou non substitué, un groupe naphtyle substitué ou non substitué, un groupe anthracenyle substitué ou non substitué, un groupe carbazolyle substitué ou non substitué, un groupe benzofuranyle substitué ou non substitué, un groupe benzothiophényle substitué ou non substitué, un groupe fluorényle substitué ou non substitué, un groupe dibenzothiophényle substitué ou non substitué, un groupe dibenzofuranyle substitué ou non substitué, ou une combinaison de ceux-ci.

7. Composition selon l'une quelconque des revendications 1 à 6, dans laquelle X² est l'un des substituants listés dans le Groupe 3 :

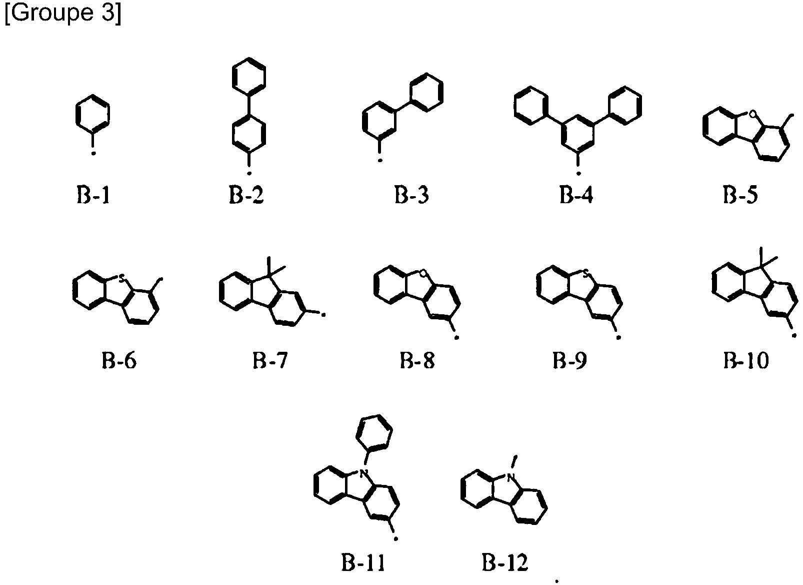

8. Composition selon la revendication 1, dans laquelle le premier composé hôte est l'un des composés listés dans le Groupe 4 : 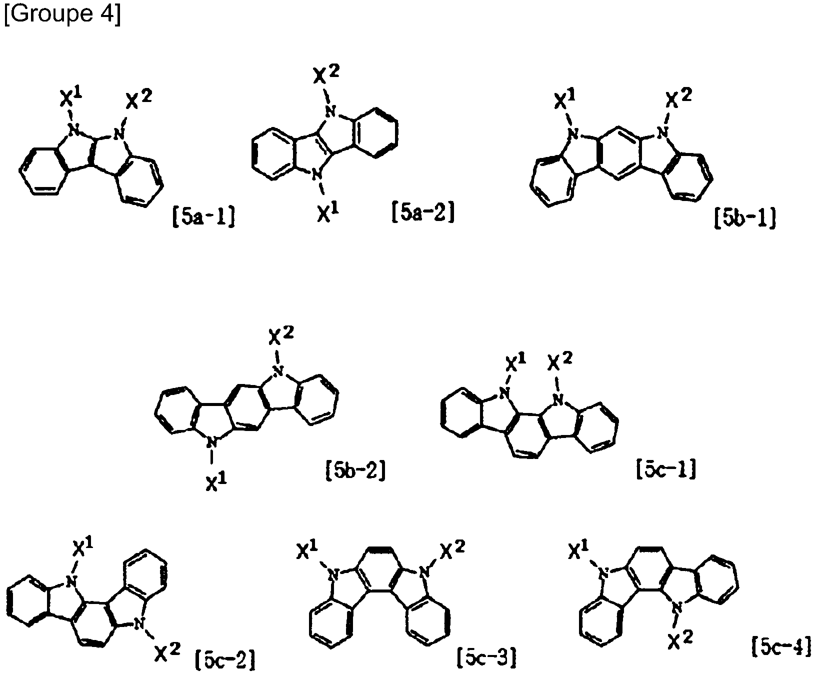 dans laquelle, dans le Groupe 4,
X¹ représente *-Y¹-ET,
X² représente *-Y²-Ar¹,
ET représente un substituant capable d'accepter un électron lorsqu'un champ électrique est appliqué,
Ar¹ représente un groupe aryle en C6 à C30 substitué ou non substitué, un groupe hétéroaryle en C2 à C30 substitué ou non substitué, ou une combinaison de ceux-ci, et
Y¹ et Y² représentent chacun indépendamment une liaison simple, un groupe alkylène en C1 à C20 substitué ou non substitué, un groupe alcénylène en C2 à C20 substitué ou non substitué, un groupe arylène en C6 à C30 substitué ou non substitué, un groupe hétéroarylène en C2 à C30 substitué ou non, ou une combinaison de ceux-ci.

9. Composition selon l'une quelconque des revendications 1 à 8, dans laquelle Ar⁴ est un groupe phényle substitué ou non substitué, un groupe biphényle substitué ou non substitué, un groupe terphényle substitué ou non substitué, un groupe naphtyle substitué ou non substitué, un groupe anthracenyle substitué ou non substitué, un groupe carbazolyle substitué ou non substitué, un groupe benzofuranyle substitué ou non substitué, un groupe benzothiophényle substitué ou non substitué, un groupe fluorényle substitué ou non substitué, un groupe dibenzofuranyle substitué ou non substitué, un groupe dibenzothiophényle substitué ou non substitué, ou une combinaison de ceux-ci.

10. Composition selon l'une quelconque des revendications 1 à 9, dans laquelle le deuxième composé hôte est l'un des composés listés dans le Groupe 6 :

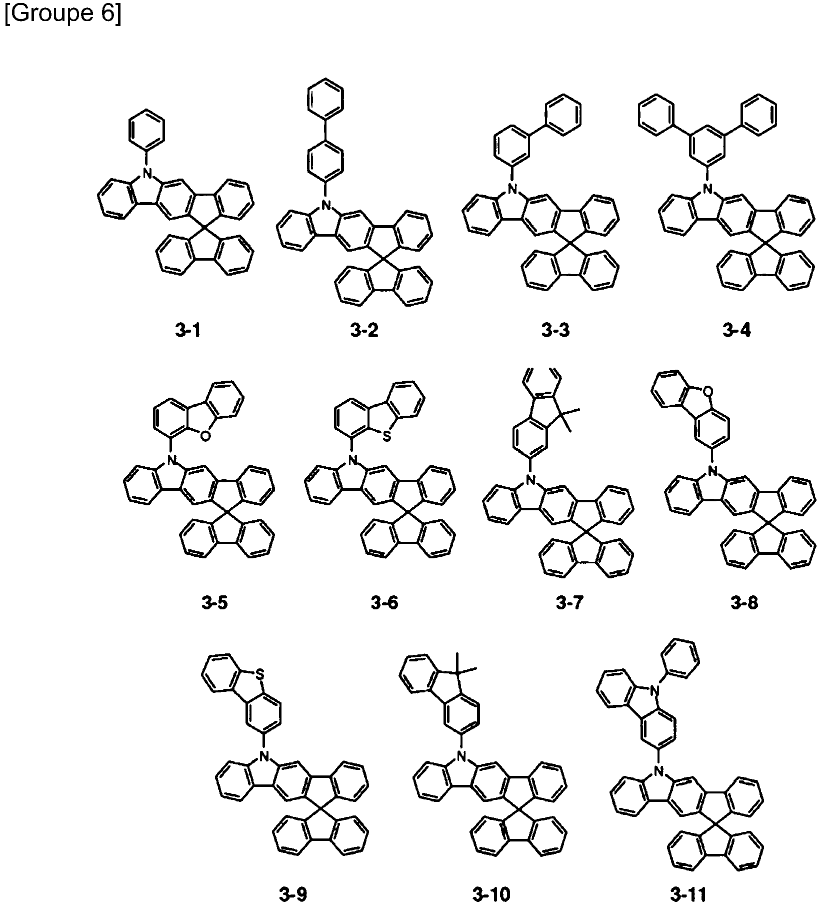

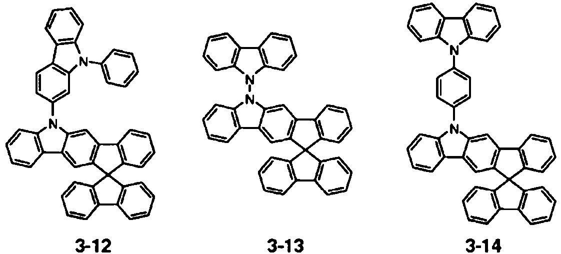

11. Composition selon l'une quelconque des revendications 1 à 10, dans laquelle Ar⁵ est un groupe phényle substitué ou non substitué, un groupe biphényle substitué ou non substitué, un groupe terphényle substitué ou non substitué, un groupe naphtyle substitué ou non substitué, un groupe anthracenyle substitué ou non substitué, un groupe carbazolyle substitué ou non substitué, un groupe benzofuranyle substitué ou non substitué, un groupe benzothiophényle substitué ou non substitué, un groupe fluorényle substitué ou non substitué, un groupe dibenzothiophényle substitué ou non substitué, un groupe dibenzofuranyle substitué ou non substitué, ou une combinaison de ceux-ci.

12. Composition selon l'une quelconque des revendications 1 à 11, dans laquelle le deuxième composé hôte est l'un des composés listés dans le Groupe 7 suivant :

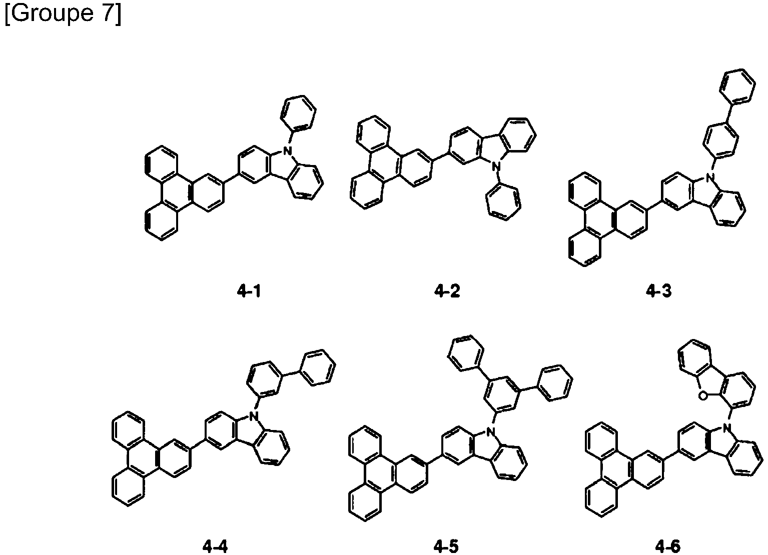

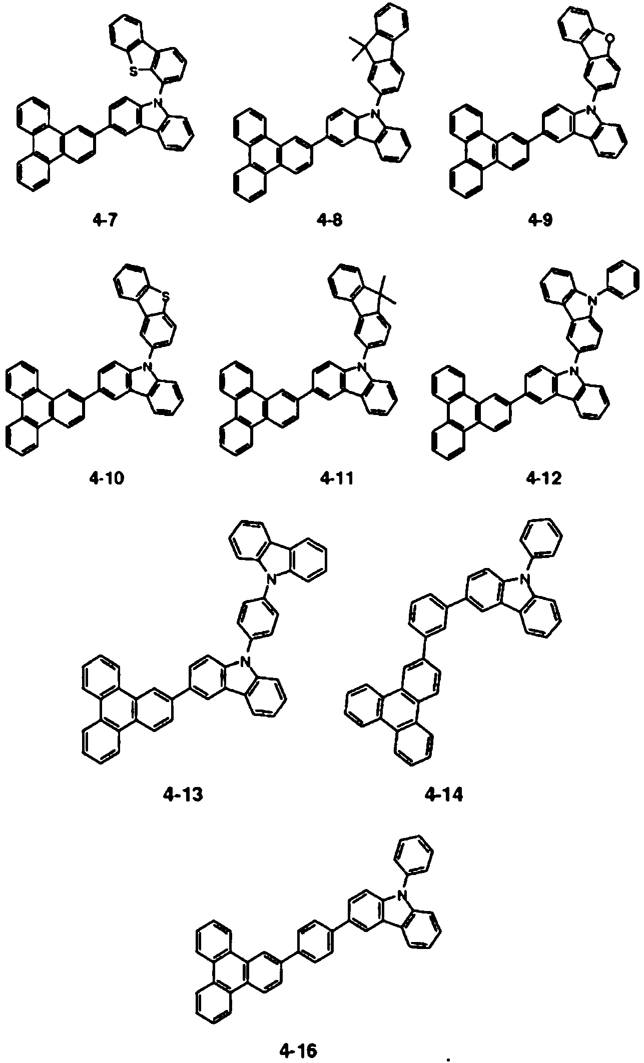

13. Composition selon l'une quelconque des revendications 1 à 12, dans laquelle le premier composé hôte et le deuxième composé hôte sont inclus dans un rapport pondéral d'environ 1:10 à environ 10:1.

14. Dispositif optoélectrique organique, comprenant
une anode et une cathode face à face l'une de l'autre,
au moins une couche organique interposée entre l'anode et la cathode,
dans lequel la couche organique comprend la composition selon l'une quelconque des revendications 1 à 13.

15. Dispositif d'affichage comprenant le dispositif optoélectrique organique selon la revendication 14.
